(19) Europäisches Patentamt
European Patent Office
Office européen des brevets

(11) Numéro de publication: **0 053 073**
**B1**

(12) # FASCICULE DE BREVET EUROPEEN

(45) Date de publication du fascicule du brevet: **12.09.84**

(51) Int. Cl.³: **C 07 D 498/04** // (C07D498/04, 265/00, 205/00)

(21) Numéro de dépôt: **81401831.3**

(22) Date de dépôt: **19.11.81**

(54) **Nouvelles oxacéphalosporines et leur préparation.**

(30) Priorité: **20.11.80 FR 8024639**

(43) Date de publication de la demande: **02.06.82 Bulletin 82/22**

(45) Mention de la délivrance du brevet: **12.09.84 Bulletin 84/37**

(84) Etats contractants désignés: **AT BE CH DE FR GB IT LI LU NL SE**

(56) Documents cités:
**FR - A - 2 137 899**
**FR - A - 2 361 399**
**FR - A - 2 385 722**
**US - A - 3 984 401**
**US - A - 4 065 620**
**US - A - 4 248 868**

**J. Med. Chem. 18 (10), 986 (1975)**
**Liebigs Ann. Chem., (1978), 362**
**J.C.S. Perkin I, (1974), 37**
**J. Org. Chem. 42 (11), 1960 (1977)**
**J.C.S. Perkin I, 1548 (1977)**
**Bull. Chem. Soc. Japan, 48 (1), 367 (1975)**
**Synthesis, 826 (1978)**
**Angew. Chem. Int. Ed. Engl., 18 (9), 687 (1978)**
**Chem. Pharm. Bull., 28 (4) 1339 (1980)**

(73) Titulaire: **RHONE-POULENC SANTE, Les Miroirs 18 Avenue d'Alsace, F-92400 Courbevoie Cedex (FR)**

(72) Inventeur: **Farge, Daniel, 30 rue des Pins Sylvestres, F-94320 Thiais (FR)**
Inventeur: **Le Roy, Pierre, 2 allée des Cerisiers, F-94320 Thiais (FR)**
Inventeur: **Moutonnier, Claude, 3 rue Auguste Rodin, F-92350 Le Plessis Robinson (FR)**
Inventeur: **Peyronel, Jean-François, 36 parc d'Ardenay, F-91110 Palaiseau (FR)**
Inventeur: **Plau, Bernard, 53 rue de Mesly, F-94000 Creteil (FR)**

(74) Mandataire: **Gaumont, Robert et al, RHONE-POULENC RECHERCHES Service Brevets Pharma 25, Quai Paul Doumer, F-92408 Courbevoie Cedex (FR)**

(56) Documents cités: (suite)
**Bull. Chem. Soc. Japan, 35, 1498 (1962)**
**J. Chem. Soc. (C), 460 (1969)**
**Org. Synth., 53, 104 (1973)**
**J. Org. Chem., 43 (2), 283 (1978)**
**J. Org. Chem., 31, 1922 (1966)**
**Chem. Ber., 54, 437 (1921)**
**Chem. Ber., 67, 710 (1934)**
**C.R. Acad. Sci., 185, 1173 (1927)**
**J. Am. Pharm. Ass., 40, 61 (1951)**
**Chem. Ber. 101, 4048 (1968)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

**Description**

Dans la demande française FR-A-2 385 722 ont été décrits les dérivés de céphalosporine et d'oxacéphalosporine de formule générale:

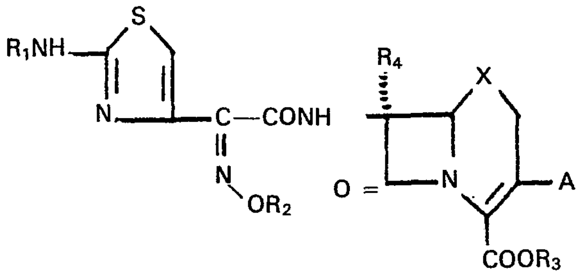

dans laquelle X est un atome de soufre ou d'oxygène et A représente notamment un radical acyloxyméthyle ou acylthiométhyle. Ces produits sont des agents antibactériens.

Dans la demande française FR-A-2 137 899 ont été décrites les céphalosporines de formule générale:

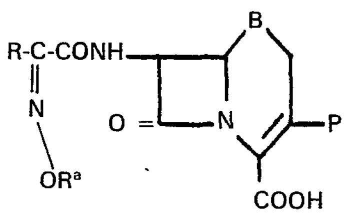

dans laquelle B est $>S$ ou $>S \rightarrow O$ et P peut être par exemple un radical de structure:

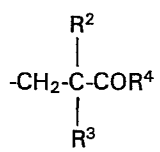

pour lequel $R^2$, $R^3$ et $R^4$ représente notamment des atomes d'hydrogène.

Ces produits sont également des agents antimicrobiens.

La présente invention concerne de nouvelles oxacéphalosporines de formule générale:

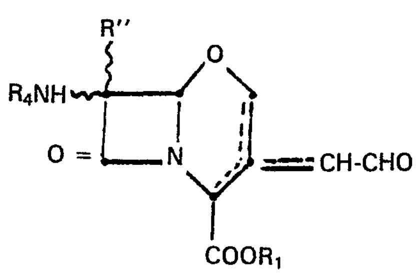

(I)

et leur préparation.

Le produit de formule générale (I) se présente sous forme bicyclooctène-2 ou -3 ou oxoéthylidène-3 bicyclooctane (selon la nomenclature des Chemical Abstracts), $R_1$ est un radical protecteur d'acide facilement éliminable et

a) le symbole $R_4$ représente un radical de formule générale:

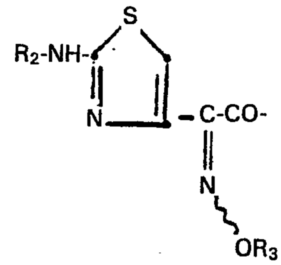

(II)

[dans laquelle $R_2$ est un radical protecteur d'amino, $R_3$ est un atome d'hydrogène, un radical protecteur d'oxime, un radical alcoyle, vinyle, ou carboxyalcoyle représenté par la formule générale:

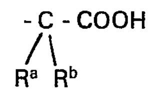

(III)

dans laquelle les radicaux $R^a$ et $R^b$, qui sont identiques ou différents, représentent des atomes d'hydrogène ou des radicaux alcoyle, ou forment ensemble un radical alcoylène contenant 2 ou 3 atomes de carbone et dont la fonction acide peut être protégée], ou $R_4$ représente un radical $\alpha$-carboxyarylacétyle libre ou protégé dans lequel aryle représente phényle, p.hydroxy phényle libre ou protégé ou thiényle-2 ou -3, et

le symbole R'' représente un atome d'hydrogène ou un radical méthoxy en position $7\alpha$, ou bien

b) le symbole $R_4$ représente un radical facilement éliminable et le symbole R'' représente un atome d'hydrogène ou un radical méthoxy en $7\alpha$ ou un atome d'hydrogène en $7\beta$.

Il est entendu que, sauf mention spéciale, les portions ou radicaux alcoyles cités ci-dessus (ou qui seront cités ci-après) sont droits ou ramifiés et contiennent 1 à 4 atomes de carbone.

Il est également entendu que les mélanges des isomères bicyclooctène-2 ou -3 ou oxoéthylidène-3 bicyclooctane entrent dans le cadre de la présente invention.

Par ailleurs, le groupement $OR_3$ du radical de formule générale (II) peut se trouver dans l'une des positions syn ou anti, et ces isomères et leurs mélanges entrent dans le cadre de la présente invention.

La forme syn peut être représentée par la formule générale:

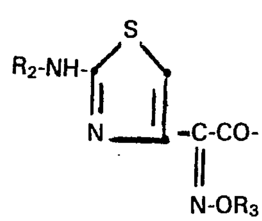

(IIa)

La forme anti peut être représentée par la formule générale:

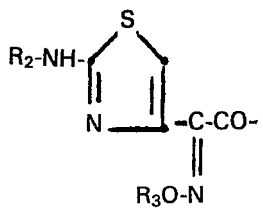

(IIb)

A titre d'exemple:

— $R_1$ peut être un radical protecteur d'acide choisi parmi méthoxyméthyle, t.butyle, benzhydryle, benzyle, nitrobenzyle ou p.méthoxybenzyle;

— $R_2$ peut être un radical protecteur d'amino choisi parmi t.butoxycarbonyle, trichloro-2,2,2 éthoxycarbonyle, trichloracétyle, trityle, benzyle, dibenzyle, benzyloxycarbonyle, p.nitrobenzyloxycarbonyle, p.méthoxybenzyloxycarbonyle, chloracétyle, formyle ou trifluoroacétyle;

— lorsque $R_3$ est un radical protecteur, il peut être choisi parmi: trityle, tétrahydropyrannyle, méthoxy-2 propyle-2, alcoyloxycarbonyle (tel que t.butoxycarbonyle) ou aryloxycarbonyle (tel que benzyloxycarbonyle)

— lorsque $R_3$ ou lorsque $R_4$ contient un radical carboxy, celui-ci peut être protégé par un radical tel que cité ci-dessus pour $R_1$

— lorsque $R_4$ contient un groupement hydroxy, ce dernier peut être protégé par un radical tel que défini ci-dessus lorsque $R_3$ est un radical protecteur

— lorsque $R_4$ est un radical facilement éliminable, il peut être choisi parmi

1) benzhydryle ou trityle,

2) un radical acyle de formule générale:

$R_5CO-$ (IVa)

dans laquelle $R_5$ représente:

a) un atome d'hydrogène, un radical alcoyle contenant 1 à 7 atomes de carbone, méthyle substitué par 1 à 3 atomes d'halogène, alcényle contenant 3 à 7 atomes de carbone ou cyanométhyle,

b) un radical phényle pouvant être jusqu'à 3 fois substitué (par des atomes d'halogène ou par des radicaux hydroxy, nitro, cyano, trifluorométhyle, alcoyle ou alcoyloxy) ou un radical thiényle-2 ou -3,

c) un radical de formule générale:

$R'_5$ Y $CH_2$- (IVb)

dans laquelle $R'_5$ représente un radical tel que défini en b) et Y représente un atome de soufre ou d'oxygène,

d) un radical arylalcoyle de formule générale:

$R''_5CH_2$- (IVc)

dans laquelle $R''_5$ représente un radical phényle pouvant être jusqu'à 3 fois substitué (par des radicaux hydroxy, alcoyle ou alcoyloxy) ou un radical hétérocyclyle tel que thiényle-2 ou -3, furyle-2 ou -3 ou tétrazolyle-1,

3) un radical de formule générale:

$R_6OCO$- (IVd)

dans laquelle $R_6$ est un radical alcoyle ramifié non substitué, un radical alcoyle droit ou ramifié portant un ou plusieurs substituants tels que des atomes d'halogène ou les radicaux cyano, phényle ou phényle substitué (par un ou plusieurs atomes d'halogène ou radicaux alcoyle, alcoyloxy, nitro ou phényle), un radical triméthylsilyl-2 éthyle, un radical vinyle ou allyle, ou un radical quinolyle,

4) un radical de formule générale:

$R_6$-S- (IVe) ou $R_6$-SE- (IVf)
$(O)_n$

dans lesquelles le reste $R_6$ est un radical alcoyle ou phényle ou phényle substitué par un ou plusieurs atomes d'halogène ou radicaux nitro ou alcoyle et n est égal à 0 ou 1,

5) ou un radical bis(nitro-4 benzyl)phosphoryle, ou bien

6) le groupe $R_4NH$- peut être remplacé par un radical alcoylaminométhylèneamino, ou par un radical de formule générale

Ar -CH = N- (IVg)

dans laquelle Ar est un groupe phényle éventuellement substitué par un ou plusieurs radicaux tels que alcoyloxy, nitro, alcoyle ou hydroxy.

Comme exemples de radicaux $R_4$ pouvant être utilisés, on peut citer les radicaux suivants:
formyle, acétyle, chloracétyle, trichloracétyle, phénylacétyle, phénoxyacétyle, benzoyle,
t.butoxycarbonyle
chloro-2 diméthyl-1,1 éthoxycarbonyle
trichloro-2,2,2 éthoxycarbonyle
trichloro-2,2,2 diméthyl-1,1 éthoxycarbonyle
cyano-2 diméthyl-1,1 éthoxycarbonyle
triméthylsilyl-2 éthoxycarbonyle
benzyloxycarbonyle
p.méthoxybenzyloxycarbonyle
diméthoxy-3,5 benzyloxycarbonyle
p.nitrobenzyloxycarbonyle
diphénylméthoxycarbonyle
(biphényl-4)-2 isopropyloxycarbonyle
vinyloxycarbonyle
allyloxycarbonyle
quinolyl-8 oxycarbonyle
o.nitrophénylthio
p.nitrophénylthio
bis(nitro-4 benzyl)phosphoryle

Comme exemples de radicaux méthylèneamino définis précédemment en 6), on peut citer:
diméthylaminométhylèneamino
diméthoxy-3,4 benzylidèneamino
nitro-4 benzylidèneamino
di t.butyl-3,5 hydroxy-4 benzylidèneamino

Selon l'invention, les produits de formule générale (I) peuvent être obtenus par hydrolyse de l'énamine (ou du mélange d'énamines isomères) de formule générale:

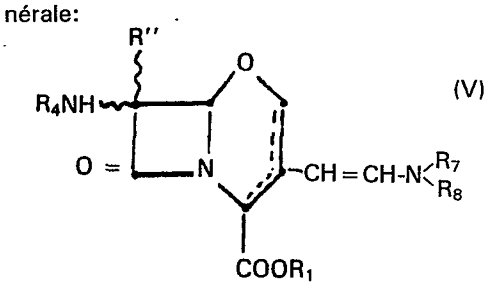

(V)

dans laquelle, R'', $R_1$ et $R_4$ étant définis comme précédemment (étant entendu que, le cas échéant, le radical carboxy contenu par $R_4$ est protégé), le produit se présente sous forme bicyclooctène-2 ou -3, le substituant sur l'atome de carbone en position -3 du bicyclooctène présente la stéréoisomérie cis ou trans (désignée ci-après respectivement par Z ou E) et $R_7$ et $R_8$ qui sont identiques ou différents représentent des radicaux alcoyle (éventuellement substitués par un radical alcoyloxy ou dialcoylamino) ou phényle, ou forment ensemble avec l'atome d'azote auquel ils sont rattachés un hétérocycle saturé à 5 ou 6 chaînons, contenant éventuellement un autre hétéroatome choisi parmi l'azote, l'oxygène ou le soufre et éventuellement substitué par un radical alcoyle.

On opère généralement dans un acide organique (acide formique, acide acétique par exemple) ou minéral (acide chlorhydrique, acide sulfurique par exemple) en présence ou non d'un solvant, en milieu aqueux ou organique, à une température comprise entre —20°C et la température de reflux du mélange réactionnel. Lorsque l'on opère en milieu organique, l'hydrolyse est réalisée par addition d'eau au mélange réactionnel, puis on traite éventuellement par une base minérale (par exemple bicarbonate alcalin) ou organique (par exemple amine tertiaire ou pyridine).

Lorsque l'on opère en présence d'un solvant, il n'est pas nécessaire que le solvant soit miscible à la phase aqueuse acide. Le contact est alors réalisé par agitation vive.

Parmi les solvants utilisables, peuvent être cités les solvants chlorés, l'acétate d'éthyle, le tétrahydrofuranne, l'acétonitrile, le diméthylformamide, les alcools. Il n'est pas absolument nécessaire d'avoir purifié l'intermédiaire de formule générale (V) pour mettre en oeuvre cette réaction.

Lorsque l'on veut obtenir un aldéhyde de formule générale (I) dans laquelle $R_4$ contient une fonction acide libre, il est nécessaire d'opérer à partir d'une énamine dans laquelle $R_1$ et le groupement protecteur de la fonction acide de $R_4$ sont différents et éliminables sélectivement.

L'élimination du radical protecteur s'effectue dans les conditions décrits ci-après.

Les produits de formule générale (V) peuvent être obtenus par action d'un produit de formule générale:

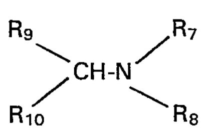
(VI)

éventuellement préparé in situ, [pour lequel $R_7$ et $R_8$ sont définis comme précédemment et $R_9$ et $R_{10}$ qui sont identiques ou différents, soit représentent des groupements de formule générale:

$$-X_2\ R_{11} \qquad \text{(VII)}$$

dans laquelle $X_2$ représente un atome d'oxygène et $R_{11}$ représente un radical alcoyle ou phényle, soit représentent l'un un radical de formule générale (VII) (dans lequel $X_2$ représente un atome d'oxygène ou de soufre et $R_{11}$ est alcoyle ou phényle), et l'autre un radical amino de formule générale:

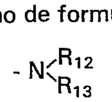
(VIII)

dans laquelle $R_{12}$ et $R_{13}$ sont définis comme $R_7$ et $R_8$, soit encore $R_9$ et $R_{10}$ représentent chacun un radical de formule générale (VIII)] sur un dérivé d'oxacéphalosporine de formule générale:

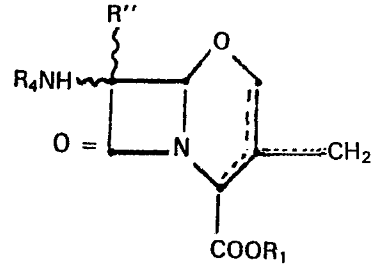
(IX)

dans laquelle, R'', $R_1$ et $R_4$ étant définis comme précédemment dans la formule générale (V), le produit se présente sous forme méthyl-3 bicyclooctène-2 ou -3 ou méthylène-3 bicyclooctane.

Lorsque l'on choisit un produit de formule générale (VI) dans laquelle le radical (VIII) est différent de $-NR_7R_8$, il est préférable de choisir un tel produit de manière que l'amine $HNR_{12}R_{13}$ soit plus volatile que $HNR_7R_8$.

On opère généralement dans un solvant organique tel qu'un amide (par exemple le diméthylformamide, le diméthylacétamide ou l'hexaméthylphosphorotriamide), un nitrile (par exemple l'acétonitrile), un ester (par exemple l'acétate d'éthyle), un éther (par exemple le dioxanne), un solvant chloré (par exemple le dichloro-1,2 éthane), ou encore dans un mélange de tels solvants à une température comprise entre 20°C et la température de reflux du mélange réactionnel.

Il est entendu que, lorsque $R_4$ représente un radical de formule générale (II) dans laquelle $R_3$ représente un atome d'hydrogène, il est préférable que l'oxime soit protégée dans les conditions décrites précédemment.

Il est également entendu que, lorsque $R_4$ contient un substituant hydroxy, il est préférable de protéger ce dernier.

La protection des radicaux s'effectue dans les conditions décrites précédemment.

L'élimination des radicaux protecteurs s'effectue dans les conditions décrites ci-après pour les produits de formule générale (XIV).

Les produits de formule générale (VI) peuvent être préparés selon les méthodes décrites par H. BREDERECK et coll., Chem. Ber. *101,* 41 (1968), Chem. Ber. *101,* 3058 (1968) et Chem. Ber. *106,* 3725 (1973).

Les dérivés de l'oxacéphalosporine de formule générale (IX) dans laquelle $R_4$ représente un radical de formule générale (II) ou un radical $\alpha$-carboxyarylacétyle dont les fonctions acide sont protégées peuvent être préparés à partir des produits de formule générale:

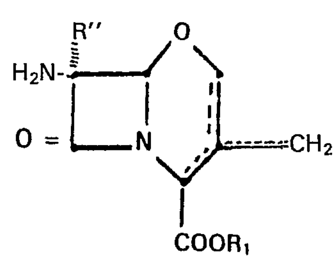
(X)

dans laquelle $R_1$ est défini comme précédemment et R'' représente un atome d'hydrogène ou un radical méthoxy en position 7$\alpha$, en opérant par action d'un acide de formule générale:

$$R_4OH \quad (XI)$$

dans laquelle $R_4$ est défini comme précédemment, ou d'un dérivé réactif de cet acide, dans les conditions décrites ci-après en II/- pour la préparation des produits de formule générale (XII).

Les oxacéphalosporines de formules générales (IX) et (X) peuvent être préparées selon ou par analogie avec les méthodes décrites dans la littérature, par exemple:

— lorsque R'' représente un atome d'hydrogène: selon les méthodes décrites par C.L. BRANCH et coll., J.C.S. Perkin I, 2268 (1979), dans la demande de brevet allemand 2 806 457, dans le brevet américain 4 108 992, dans la demande de brevet allemand 2 355 209, puis éventuellement mise en place du radical $R_4$ [lorsque l'on veut obtenir un produit de formule générale(IX)], par analogie avec les méthodes employées en chimie des céphalosporines et par exemple:

— lorsque $R_4$ est un radical formyle: selon J.C. SHEEHAN et coll., J. Amer. Chem. Soc. *80* 1154 (1958),

— lorsque $R_4$ est acétyle, chloracétyle, trichloracétyle, phénylacétyle, phénoxyacétyle ou benzoyle: selon E.H. FLYNN, Cephalosporins and Penicillins, Ac. Press (1972),

— lorsque $R_4$ est un radical t.butoxycarbonyle: selon L. MORODER et coll., Hoppe Seyler's Z. Physiol. Chem. *357* 1651 (1976),

— lorsque $R_4$ est trichloro-2,2,2 diméthyl-1,1 éthoxycarbonyl: selon J. UGI et coll., Angew. Chem. Int. Ed. Engl. *17(5)* 361 (1978),

— lorsque $R_4$ est trichloro-2,2,2 éthoxycarbonyle, chloro-2 diméthyl-1,1 éthoxycarbonyle, cyano-2 diméthyl-1,1 éthoxycarbonyle, triméthylsilyl-2 éthoxycarbonyle, benzyloxycarbonyle, p.méthoxybenzyloxycarbonyle, diméthoxy-3,5 benzyloxycarbonyle, p.nitrobenzyloxycarbonyle, vinyloxycarbonyle: par action d'un chloroformiate en milieu hydroorganique en présence d'un bicarbonate alcalin, ou selon le brevet belge 788 885,

— lorsque $R_4$ est diphénylméthoxycarbonyle: par action de l'azidoformiate correspondant en milieu hydroorganique, en présence d'un bicarbonate alcalin,

— lorsque $R_4$ est (biphényl-4)-2 isopropyloxycarbonyle: par analogie avec la méthode décrite dans Helv. Chim. Acta, *51* 924 (1968),

— lorsque $R_4$ est quinolyl-8 oxycarbonyle ou allyloxycarbonyle: par action du carbonate correspondant en milieu hydroorganique basique,

— lorsque $R_4$ est o.nitrophénylthio ou p.nitrophénylthio: par analogie avec la méthode décrite par T. KOBAYASHI et coll., Chem. Pharm. Bull *27(11)* 2718 (1979),

— lorsque $R_4NH$ est remplacé par diméthylaminoéthylèneamino: par analogie avec la méthode décrite par J.F. FITT, J. Org. Chem. *42(15)* 2639 (1977),

— lorsque $R_4NH$ est remplacé par nitro-4 benzylidèneamino ou diméthoxy-3,4 benzylidèneamino: selon la méthode décrite par R.A. FIRESTONE, Tetrahedron Lett., 375 (1972),

— lorsque $R_4NH$ est remplacé par di t.butyl-3,5 hydroxy-4 benzylidèneamino: selon la méthode décrite par H. YANAGISAWA et coll., Tetrahedron Lett., 2705 (1975),

— lorsque $R_4$ est bis(nitro-4 benzyl)phosphoryle: selon la méthode décrite dans la demande de brevet japonais 77 125 185,

— lorsque R'' représente un radical méthoxy: par analogie avec les méthodes décrites ci-après pour la préparation des produits de formule générale (XXI) ou (XXIIa) ou selon la demande de brevet allemand 2 806 457 puis, lorsque l'on veut obtenir un produit de formule générale (IX) mise en place du radical $R_4$ par analogie avec les méthodes citées ci-dessus.

Les produits de formule générale (X) peuvent être préparés selon la méthode décrite dans le brevet belge 850 662, ou par application de la méthode décrite dans le brevet belge 877 884, lorsque $R_4$ est un radical de formule générale (II) dans laquelle $R_3$ est hydrogène ou alcoyle.

Les produits de formule générale (X) peuvent être préparés selon la méthode décrite dans le brevet belge 869 079 lorsque $R_4$ est un radical de formule générale (II) dans laquelle $R_3$ est vinyle.

Les produits de formule générale (X) peuvent être préparés selon les méthodes décrites dans les brevets belges 864 810, 865 298, 876 541 et 876 542 lorsque $R_4$ est un radical de formule générale (II) dans laquelle $R_3$ est un substituant de formule générale (III).

Les produits de formule générale (X), lorsque $R_4$ est un radical $\alpha$-carboxyarylacétyle, peuvent être préparés selon les méthodes suivantes:

— lorsque le groupement aryle représente un groupement p.hydroxy phényle: selon la méthode décrite dans la demande de brevet japonais 79 106 447 ou dans le brevet belge 852 912,

— lorsque le groupement aryle représente un groupement thiényle-2: selon D. IVANOV et N. MAREKOV: Compt. Rend. Acad. Bulgare Sci., *8(11&* 29 (1955),

— lorsque le groupement aryle représente un groupement thiényle-3: selon le brevet britannique 1 125 557.

Les nouveaux produits de formule générale (I) sont utiles comme intermédiaires pour la préparation de thiovinyl oxacéphalosporines de formule générale:

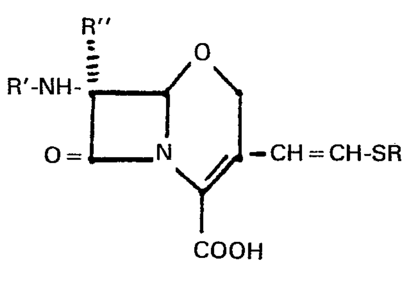

(XII)

dans laquelle le symbole R est choisi parmi les significations:

1) pyridyle-2,-3 ou -4 éventuellement N-oxydés
2) pyrimidinyle-2

3) méthyl-6 oxyde-1 pyridazinyle-3

4) dioxo-5,6 tétrahydro-1,4,5,6 triazine-1,2,4 yle-3 substitué en position -4 par

a) un radical alcoyle contenant 1 ou 2 atomes de carbone éventuellement substitué par un radical alcoyloxy, alcoylthio, formyle,

b) un radical allyle, dihydroxy-2,3 propyle, dihydroxy-1,3 propyle-2 ou formyl-2 hydroxy-2 éthyle,

c) un radical alcoyle contenant 2 ou 3 atomes de carbone substitué par hydroxy, carbamoyloxy, acyloxy ou acylamino (dont les portions acyles sont non-substituées ou substituées par amino), alcoylsulfonylamino, uréido, alcoyluréido ou dialcoyluréido.

5) dioxo-5,6 tétrahydro-1,4,5,6 triazine-1,2,4 yle-3 substitué en position -1 ou dioxo-5,6 tétrahydro-1,2,5,6 triazine-1,2,4 yle-3 substitué en position -2 par un radical alcoyle contenant 1 ou 2 atomes de carbone éventuellement substitué par un radical formyle

6) alcoyl-2 dihydro-2,5 oxo-5 triazine-1,2,4 yle-3 éventuellement substitué en position -6 par un radical alcoyle, alcoyloxy dont les portions et radicaux alcoyles contiennent 1 ou 2 atomes de carbone

7) amino-1 dihydro-1,2 oxo-2 pyrimidinyle-4

8) thiadiazol-1,3,4 yle-5 substitué par alcoyle, dialcoylaminoalcoyle ou alcylaminoalcoyle, ou

9) tétrazolyle-5 substitué en position -1 par

a) un radical alcoyle contenant 1 ou 2 atomes de carbone éventuellement substitué par un radical formyle,

b) un radical alcoyle contenant 2 ou 3 atomes de carbone substitué par hydroxy, acylamino, ou dialcoylamino, ou

c) par un radical dihydroxy-2,3 propyle ou dihydroxy-1,3 propyle-2

10)a) alcoyl-1 triazol-1,2,4 yle-5 éventuellement substitué en position -3 par un radical alcoyloxycarbonyle dont les radicaux alcoyle et alcoyloxy contiennent 1 ou 2 atomes de carbone,

b) alcoyl-1 triazol-1,3,4 yle-5,

le symbole R' représente un radical de formule générale (II) dans laquelle $R_2$ est un atome d'hydrogène et $R_3$ est un atome d'hydrogène, un radical alcoyle, vinyle ou carboxyalcoyle de formule générale (III) dans laquelle $R^a$ et $R^b$ sont définis comme précédemment, ou R' représente un radical $\alpha$-carboxyarylacétyle dans lequel aryle représente phényle, p.hydroxyphényle, ou thiényle-2 ou -3 et le symbole R'' est un atome d'hydrogène ou un radical méthoxy en $7\alpha$.

Il est entendu que le substituant en position -3 des produits de formule générale (XII) peut se présenter sous forme E ou Z.

I/- Les thiovinyl-3 oxacéphalosporines de formule générale (XII) peuvent être préparées en opérant de la manière suivante:

A) - On fait agir un dérivé activé des acides $R'_{14}SO_3H$ ou $R''_{14}COOH$, du type:

| | | |
|---|---|---|
| $(R'_{14}SO_2)O$ | (a) | |
| $R'_{14}SO_2Hal$ | (b) | (XII) |
| $(R''_{14}CO)_2O$ | (c) | |
| $R''_{14}COHal$ | (d) | |

[dans lesquelles $R'_{14}$ est un radical alcoyle, trifluorométhyle, trichlorométhyle ou un radical phényle éventuellement substitué par un atome d'halogène ou par un radical alcoyle ou nitro, et $R''_{14}$ est défini comme $R'_{14}$ ou représente un radical alcylméthyle, acyl-2 éthyle, acyl-2 propyle, alcoyloxycarbonylméthyle, alcoyloxycarbonyl-2 éthyle, ou alcoyloxycarbonyl-2 propyle et Hal représente un atome d'halogène],

ou un agent d'halogénation,

sur un produit de formule générale (I), ou sur un mélange de ses isomères, puis élimine éventuellement les radicaux protecteurs, pour obtenir un produit de formule générale:

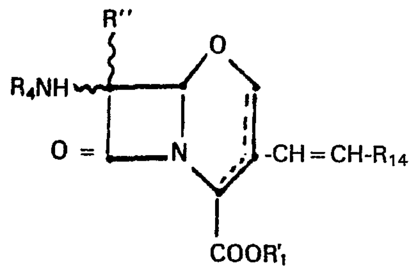

(XIV)

qui se présente sous forme bicyclooctène-2 ou -3, et dans laquelle

$a_1$) le symbole $R'_1$ est un atome d'hydrogène ou un radical protecteur tel que défini précédemment pour $R_1$,

le symbole $R_4$ est défini comme précédemment pour les produits de formule générale (I) en a), ou représente un radical de formule générale (II) dans laquelle le radical $R_2$ représente un atome d'hydrogène, et le symbole R'' est défini comme précédemment en a), ou bien

$b_1$) le symbole $R'_1$ est défini comme $R_1$ précédemment,

les symboles $R_4$ et R'' sont définis comme précédemment en b), et

le symbole $R_{14}$ est un radical de formule générale:

$R'_{14}SO_2O-$ (XVa)

$R''_{14}COO-$ (XVb)

dans lesquelles $R'_{14}$ et $R''_{14}$ sont définis comme ci-dessus, ou $R_{14}$ est un atome d'halogène choisi parmi le chlore, le brome et l'iode,

et dont le substituant en position -3 du bicyclooctène présente la stéréoisomérie E ou Z.

L'orsque l'on désire mettre en oeuvre un produit de formule générale (I) dans laquelle $R_4$ est un radical de formule générale (II) dont le reste $R_3$ est un atome d'hydrogène, il est nécessaire de protéger préalablement l'oxime.

Lorsque l'on désire mettre en oeuvre un aldéhyde de formule générale (I) dans laquelle $R_4$ contient un groupement carboxy, ce radical peut être libre ou protégé si l'on fait agir un dérivé activé des acides $R'_{14}SO_3H$ ou $R''_{14}COOH$; par contre il est nécessaire de le protéger préalablement si l'on fait agir un agent d'halogénation.

Lorsque l'on désire mettre en oeuvre un aldéhyde dans lequel le radical $R_4$ est $\alpha$-carboxy (p.hydroxyphényl)acétyle, il est nécessaire de protéger le radical hydroxy.

La protection des radicaux s'effectue dans les conditions décrites précédemment.

On opère généralement en présence d'une base tertiaire de formule générale:

$$X_1 - N\begin{matrix}Y_1\\Z_1\end{matrix} \qquad (XVI)$$

dans laquelle $X_1$, $Y_1$ et $Z_1$ représentent des radicaux alcoyle ou phényle, ou éventuellement deux d'entre eux forment un cycle avec l'atome d'azote auquel ils sont rattachés, par exemple la triéthylamine ou la NH-diméthylaniline dans un solvant organique chloré (par exemple dichlorométhane), dans un ester (acétate d'éthyle), dans un éther (par exemple dioxanne, tétrahydrofuranne), dans un amide (par exemple diméthylacétamide, diméthylformamide) dans l'acétonitrile ou la N-méthylpyrrolidone ou dans un mélange de tels solvants, ou directement dans un solvant basique comme la pyridine, ou bien lorsque $R_{14}$ est autre qu'un atome d'halogène, on peut opérer en milieu hydroorganique en présence d'un agent alcalin de condensation (par exemple bicarbonate alcalin, soude ou potasse), à une température comprise entre —78°C et la température de reflux du mélange réactionnel.

Eventuellement on opère sous azote.

Il n'est pas absolument nécessaire d'avoir purifié le produit de formule générale (I) pour le mettre en oeuvre dans cette réaction.

Lorsque l'on veut préparer un produit de formule générale (XIV) dans laquelle $R_{14}$ est un atome d'halogène, l'agent d'halogénation peut être choisi parmi des dérivés halogénés du phosphore, notamment:

— les composés d'addition d'halogène et de triarylphosphite, ou bien

— le trichlorure de phosphore, l'oxychlorure de phosphore, le pentachlorure de phosphore, le dichlorotriphénylphosphorane ou le catéchyltrichlorophosphorane lorsque $R_{14}$ est un atome de chlore, ou

— le tribromure de phosphore, l'oxybromure de phosphore, le pentabromure de phosphore ou le catéchyltribromophosphorane lorsque $R_{14}$ est une atome de brome.

Le catéchyltrichloro (ou tribromo) phosphorane, qui peut être préparé in situ, peut être obtenu selon la méthode décrite par H. GROSS et U. KARSCH, J. Prakt. Chem, *29*, 315 (1965).

Les composés d'addition d'halogène et de triarylphosphite, qui peuvent être préparés in situ, sont décrits par H.N. RYDON et B.L. TONGE,J. Chem. Soc., 3043 (1956), par J. MICHALSKI et coll., J. Org. Chem., *45*, 3122 (1980) ou dans le brevet belge 881 424 et peuvent être préparés selon les méthodes mentionnées dans ces documents.

La préparation des dérivés halogénés de formule générale (XIV) s'effectue en milieu anhydre.

Lorsque l'on veut préparer un produit de formule générale (XIV), dans laquelle $R_{14}$ est un atome de chlore ou de brome, selon les conditions opératoires on peut isoler l'intermédiaire dihalogéné de formule générale:

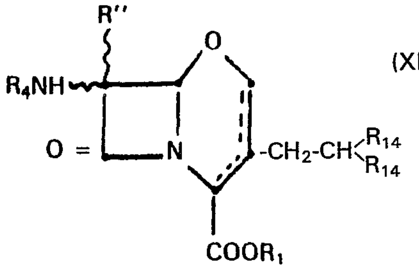

(XIVa)

[dans laquelle R'', $R_4$ et $R_1$ sont définis comme pour le produit de formule générale (I) utilisé et $R_{14}$ est défini comme ci-dessus, le produit présentant la même isomérie que le produit de formule générale (XIV)] qui est ensuite déhydrohalogéné.

Lorsque l'on veut isoler l'intermédiaire dihalogéné, on opère par action d'un agent d'halogénation, dans un solvant organique tel qu'un solvant chloré (par exemple dichlorométhane, chloroforme, tétrachlorure de carbone, dichloro-1,2 éthane), un éther (par exemple éther éthylique, oxyde de propylène, tétrahydrofuranne, dioxanne), un amide (par exemple diméthylacétamide, diméthylpropionamide, diméthylformamide, N-acétylmorpholine, N-acétylpipéridine, N-méthylpyrrolidone) ou un mélange de tels solvants, à une température un peu moins élevée que pour préparer le dérivé halogénovinyle correspondant, c'est-à-dire comprise entre —78 et 30°C.

Il est également possible d'opérer en présence d'une base telle que la pyridine dans un solvant cité ci-dessus, à une température comprise entre —78 et 0°C.

La déhydrohalogénation s'effectue en présence d'une base tertiaire telle que définie précédemment, d'une amine aromatique (par exemple, pyridine, picoline, quinoléine) ou d'une base minérale (telle que la soude, la potasse, un carbonate ou un bicarbonate alcalin ou alcalino-terreux), en milieu organique ou hydroorganique dans les solvants cités précédemment, à une température comprise entre —20°C et la température de refluc du mélange réactionnel.

Il n'est pas absolument nécessaire d'avoir purifié l'intermédiaire dihalogéné pour procéder à sa déhydrohalogénation.

L'élimination des différents radicaux protecteurs peut s'effectuer simultanément ou successivement.

A titre d'exemple,

1/ l'élimination des groupements protecteurs d'amines s'effectue

— lorsqu'il s'agit d'un radical t.butoxycarbonyle, trityle, p.méthoxybenzyloxycarbonyle ou formyle: par traitement en milieu acide. On utilise par exemple l'acide trifluoracétique en opérant à une température comprise entre 0 et 20°C, ou bien on utilise l'acide formique, phosphorique ou polyphosphorique purs ou en présence d'eau à une température comprise entre 20 et 60°C, ou encore l'acide p.toluènesulfonique ou méthanesulfonique dans l'acétone ou l'acétonitrile, à une température comprise entre 20°C et la température de reflux du mélange réactionnel. Dans ces conditions lorsque $R_4$ représente un radical de formule générale (II) le produit de formule générale (XIV) peut être obtenu sous forme de trifluoroacétate, de solvate avec l'acide formique, de phosphate, de méthanesulfonate ou de p.toluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique;

— lorsqu'il s'agit d'un radical trichloro-2,2,2 éthoxycarbonyle ou p.nitrobenzyloxycarbonyle: par réduction (notamment traitement par le zinc dans l'acide acétique);

— lorsqu'il s'agit d'un radical chloracétyle ou trichloracétyle: par application de la méthode décrite dans le brevet français publié sous le n° 2 243 199;

— lorsqu'il s'agit d'un radical benzyle, dibenzyle ou benzyloxycarbonyle: par hydrogénation catalytique;

— lorsqu'il s'agit d'un radical trifluoracétyle: par traitement en milieu basique.

2/ L'élimination des groupements protecteurs du radical carboxy s'effectue:

— lorsqu'il s'agit d'un groupement t.butyle, p.méthoxybenzyle ou benzhydryle: par traitement en milieu acide, dans les conditions décrites ci-dessus pour l'élimination du radical trityle protecteur d'amino. Dans le cas du radical benzhydryle, on peut opérer en présence d'anisole;

— lorsqu'il s'agit d'un groupement méthoxyméthyle: par traitement en milieu acide dilué;

— lorsqu'il s'agit d'un groupement nitrobenzyle: par réduction (notamment traitemment par le zinc dans l'acide acétique ou hydrogénolyse).

3/ L'élimination des groupements protecteurs de l'oxime et/ou des radicaux hydroxy s'effectue

— lorsqu'il s'agit de groupement trityle ou tétrahydropyrannyle: par acidolyse, par exemple par l'acide trifluoracétique, l'acide formique aqueux ou non, ou l'acide p.toluènesulfonique,

— lorsqu'il s'agit du groupement méthoxy-2 propyle-2: selon la méthode décrite dans le brevet belge 875 379,

— lorsqu'il s'agit des groupements alcoyloxycarbonyle ou aryloxycarbonyle: selon les méthodes décrites dans le brevet belge 871 213.

B) - Eventuellement on élimine le radical protecteur $R_4$ (ou, simultanément ou successivement, les groupements protecteurs $R_4$ et $R'_1$) d'un produit de formule générale (XIV) tel que défini en $b_1$) et dont le radical R'' est un atome d'hydrogène ou un radical méthoxy en position 7α, pour obtenir un produit de formule générale:

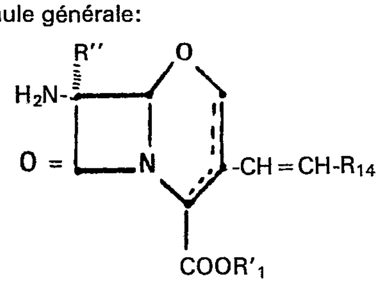

(XVII)

dans laquelle $R_{14}$ est défini comme pour la formule générale (XIV), R'' est défini comme ci-dessus et $R'_1$ est un atome d'hydrogène ou un radical protecteur tel que défini précédemment pour $R_1$.

L'élimination du radical protecteur $R_4$ s'effectue par toute méthode connue pour libérer une fonction amine sans toucher au reste de la molécule.

A titre d'exemple, on peut citer les méthodes suivantes:

— lorsque $R_4$ représente trityle, benzhydryle, trichloracétyle, chloracétyle, t.butoxycarbonyle, trichloréthoxycarbonyle, benzyloxycarbonyle, p.-methoxybenzyloxycarbonyle et p.nitrobenzyloxycarbonyle: selon les méthodes citées ci-dessus pour la libération du racidal amino du produit de formule générale (XIV);

— lorsque $R_4$ représente formyle, chloro-2 diméthyl-1,1 éthoxycarbonyle, cyano-2 dimethyl-1,1 éthoxycarbonyle, diméthoxy-3,5 benzyloxycarbonyle, diphénylméthoxycarbonyle, (biphényl-4)-2 isopropyloxycarbonyle, vinyloxycarbonyle, allyloxycarbonyle, quinolyl-8 oxycarbonyle, o.nitrophénylthio, p.nitrophénylthio, et lorsque $R_4NH$- est remplacé par diméthylaminométhylèneamino, diméthoxy-3,4 benzylidèneamino ou nitro-4 benzylidèneamino: par acidolyse;

— lorsque $R_4$ représente di t.butyl-3,5 hydroxy-4 benzylidèneamino: par traitement par le réactif T de Girard, par analogie avec la méthode décrite dans le brevet belge 863 998;

— lorsque $R_4$ représente trichloro-2,2,2 éthyle ou trichloro-2,2,2 diméthyl-1,1 éthoxycarbonyle: par traitement par le zinc dans l'acide acétique,

— lorsque $R_4$ représente acétyle, benzoyle, phénylacétyle ou phénoxyacétyle: selon la méthode décrite dans le brevet belge BE 758 800 ou selon la méthode décrite par YOSHIOKA, Tet. Letters 351 (1980),

— lorsque $R_4$ représente triméthylsilyléthoxycarbonyle: selon la méthode décrite par H. GERLACH, Helv. Chim. Acta *60* (8), 3039 (1977),

— lorsque $R_4$ représente p.nitrobenzyloxycarbonyle et benzyle: par hydrogénolyse en présence de palladium;

— lorsque $R_4$ représente bis(nitro-4 benzyl) phosphoryle: par application de la méthode décrite dans la demande de brevet japonais 77 125 185.

Lorsque R'' est un groupe méthoxy ou pourra, de préférence aux méthodes précédentes, utiliser les méthodes suivantes:

1) Dans le cas où $R_4$ représente benzhydryle ou trityle: hydrogénolyse en présence de palladium.

2) Dans le cas où $R_4$ représente des radicaux de formules générales (IVe) ou (IVf): par les méthodes décrites par E.M. GORDON, J. Am. Chem. Soc. *102*(5), 1960 (1980); T. KOBAYASHI, Bull. Chem. Soc. Japan *52*(11), 3366 (1979) et T. KOBAYASHI, Chem. Pharm. Bull. *27*, 2718 (1979).

3) Dans le cas où $R_4$ représente un radical de formule générale (IVg): par acidolyse ou par traitement par le réactif T de Girard par analogie avec la méthode décrite dans le brevet belge 863 998.

Puis on acyle le dérivé d'oxacéphalosporine de formule générale (XVII), par action d'un acide de formule générale (XI) dont la fonction amine est préalablement protégée lorsque $R_4$ est un radical de formule générale (II), ou par action d'un de ses dérivés réactifs, et on élimine éventuellement les radicaux protecteurs pour obtenir une oxacéphalosporine de formule générale (XIV) dans laquelle $R'_1$, $R_{14}$, $R_4$ et R'' sont définis comme précédemment en $a_1$).

La réaction s'effectue dans les conditions décrites ci-après pour l'action d'un acide de formule générale (XXIII) ou d'un de ses dérivés réactifs sur une amino-7 oxacéphalosporine de formule générale (XXIIa).

Le cas échéant l'élimination des radicaux protecteurs s'effectue dans les conditions décrites précédemment.

C) - On prépare alors les produits de formule générale (XII) en faisant agir un thiol de formule générale:

R - SH (XVIII)

(ou un de ses sels alcalins ou alcalino-terreux) [dans laquelle R, qui est défini comme pour les produits de formule générale (XII), est protégé à l'état d'acétal (tel que défini ci-dessous) lorsque l'on veut obtenir une oxacéphalosporine de formule générale (XII) dans laquelle R contient un radical formyle], sur un produit de formule générale (XIV) dans laquelle $R'_1$, $R_{14}$, $R_4$ et R'' sont définis comme précédemment en $a_1$) (ou sur un mélange des isomères de ce produit), puis élimine le cas échéant les radicaux protecteurs.

Lorsque l'on veut obtenir un produit de formule générale (XII) dans laquelle R contient un radical formylalcoyle, le radical R du thiol de formule générale (XVIII) est protégé à l'état d'acétal, sous forme d'un radical de formules générales:

$$\text{-alk-CH}\begin{matrix}\diagup X^{\alpha}R^{\alpha} \\ \diagdown Y^{\alpha}R^{\alpha}\end{matrix} \qquad \text{(XIXa)} \qquad \text{ou}$$

$$\text{-CH}_2\text{-CHOH-CH}\begin{matrix}\diagup X^{\alpha}R^{\alpha} \\ \diagdown Y^{\alpha}R^{\alpha}\end{matrix} \qquad \text{(XIXb)}$$

dans lesquelles alk est un radical alcoylène contenant 1 à 4 atomes de carbone, $X^{\alpha}$ et $Y^{\alpha}$ sont identiques et représentent des atomes d'oxygène ou de soufre et $R^{\alpha}$ représente un radical alcoyle, ou bien $X^{\alpha}$ et $Y^{\alpha}$ sont identiques ou différents et représentent des atomes d'oxygène ou de soufre et les radicaux $R^{\alpha}$ forment ensemble un radical alcoylène contenant 2 ou 3 atomes de carbone.

Lorsque le radical R du produit de formule générale (XVIII) est susceptible d'interférer avec la réaction, il est préférable de protéger ce groupement par exemple par un radical protecteur défini précédemment (notamment lorsque R contient un radical amino, alcoylamino, hydroxy ou carbocy).

Les radicaux dihydroxy-2,3 propyle ou dihydroxy-1,3 propyle-2 peuvent être (ou sont) protégés sous forme d'un radical diméthyl-2,2 dioxolannyl-4 méthyle ou diméthyl-2,2 dioxannyle-5.

Lorsque le radical $R_4$ représente un groupe de formule générale (II), le radical amino est libre ou protégé, lorsque $R_3$ représente un atome d'hydrogène, l'oxime est de préférence protégée et lorsque $R_3$ contient un groupement carboxy celui-ci est libre ou protégé.

Lorsque le radical $R_4$ représente un groupe $\alpha$-carboxyarylacétyle, il est préférable de protéger le radical hydroxy lorsque le substituant aryle représente p.hydroxyphényle; le groupement carboxy peut être libre ou protégé.

L'élimination de tous ces radicaux s'effectue dans les conditions décrites précédemment.

L'élimination du radical protecteur de R est effectuée avant, simultanément ou après l'élimination des autres radicaux protecteurs.

Le déblocage des groupements de formule générale (XIX a ou b) (lorsque l'on veut obtenir un produit de formule générale (XII) dans laquelle R contient un radical formylalcoyle), s'effectue:

— en présence d'un acide sulfonique (par exemple acide méthanesulfonique ou acide p.toluènesulfonique) dans un solvant organique (par exemple acétonitrile ou acétone), éventuellement en présence d'eau et éventuellement en présence d'un réactif acétalisable tel que l'acétone, l'acide glyoxylique, le benzaldéhyde ou l'acide pyruvique, à une température comprise entre 20°C et la température de reflux du mélange réactionnel;

— ou bien, lorsque le radical R est un radical dioxo-5,6 tétrahydro-1,4,5,6 triazine-1,2,4 yle-3, par action d'acide formique aqueux (contenant de préférence moins de 10% d'eau), soit en présence ou non de silice, soit par transacétalisation en présence d'un réactif acétalisable tel que défini ci-dessus.

La liberation des radicaux dihydroxy-2,3 propyle ou dihydroxy-1,3 propyle-2 protégés sous forme de radicaux diméthyl-2,2 dioxolannyl-4 méthyle ou diméthyl-2,2 dioxannyle-5, s'effectue par acidolyse, par exemple par l'acide trifluoracétique, l'acide formique aqueux ou non ou l'acide p.toluènesulfinique.

Lorsqu'on utilise l'acide formique, aqueux ou non, la libération des radicaux hydroxy protégés à l'état d'acétal cyclique peut conduire au moins partiellement aux mono ou diesters correspondants, qui peuvent être séparés le cas échéant par chromatographie.

La réaction du thiol de formule générale (XVIII) sur le dérivé d'oxacéphalosporine de formule générale (XIV) s'effectue généralement en présence d'une base organique, telle qu'une pyridine ou une base organique tertiaire de formule générale (XVI).

On utilise par exemple la diisopropyléthylamine ou la diéthylphénylamine.

Lorsque l'on fait agir un sel alcalin ou alcalinoterreux du thiol de formule générale (XVIII), il n'est pas nécessaire d'opérer en présence d'une base organique telle que défini ci-dessus.

La réaction s'effectue avantageusement dans un solvant organique tel que le diméthylformamide, le tétrahydrofuranne ou l'acétonitrile ou un mélange de tels solvants.

Il est également possible d'opérer en présence de bicarbonate alcalin dans un solvant tel que cité cidessus, éventuellement en présence d'eau.

On opère à une température comprise entre —20°C et la température de reflux du mélange réactionnel, la température choisie étant variable selon le thiol employé. De même, selon le thiol employé, la durée de réaction peut varier de 5 minutes à 48 heures.

Eventuellement on opère sous azote.

De préférence, lorsque l'on veut utiliser un bicyclooctène-3 de formule générale (XIV), on met en oeuvre un tel produit pour lequel $R'_1$ est autre que l'hydrogène.

Les thiols de formule générale (XVIII), qui peuvent être mis en oeuvre sous leur forme tautomère, peuvent être préparés par application de l'une des méthodes suivantes selon la signification du radical R:

— lorsque R est un radical pyridyle-3: selon la méthode décrite par H.M. WUEST et E.H. SAKAL, J. Am. Chem. Soc., *73*, 1210 (1951),

— lorsque R est un radical oxyde-1 pyridyle-3: selon la méthode décrite par B. BLANK et coll., J. Med. Chem. *17*, 1065 (1974),

— lorsque R est un radical oxyde-1 pyridyle-4: selon la méthode décrite par R.A.Y. JONES et coll., J. Chem. Soc. 2937 (1960),

— lorsque R est un radical méthyl-6 oxyde-1 pyridazinyle-3: selon la méthode décrite dans le brevet belge 787 635,

— lorsque R est

1°) un radical dioxo-5,6 tétrahydro-1,4,5,6 triazine-1,2,4 yle-3 substitué en position -4 par un radical $R^{\gamma}$ choisi parmi:

a) un radical allyle, alcoyle (1 ou 2 atomes de carbone), lui-même éventuellement substitué par un radical alcoyloxy ou alcoylthio,

b) un radical dihydroxy-2,3 propyle ou dihydroxy-1,3 propyle-2 (éventuellement protégé sous forme d'acétal cyclique)

c) un radical alcoyle (2 ou 3 atomes de carbone) lui-même substitué [par hydroxy, carbamoyloxy, dialcoylamino, alcoylsulfonylamino, acylamino (éventuellement substitué), uréido, alcoyluréido ou dialcoyluréido]

d) un radical de formule générale (XIXa) ou (XIXb)

2°) un radical dioxo-5,6 tétrahydro-1,4,5,6 triazine-1,2,4 yle-3 substitué en position -1, ou dioxo-5,6 tétrahydro-1,2,5,6 triazine-1,2,4 yle-3 substitué en position -2 par un radical alcoyle contenant 1 ou 2 atomes de carbone ou par un radical de formule générale (XIXa): en faisant agir un oxalate d'alcoyle sur une thiosemicarbazide de formules générales:

$R^{\gamma}$ NH CS NH-$NH_2$ (XXa)

$H_2N$ CS NH NH-$R^{\gamma'}$ (XXb)

$H_2NCSN$-$NH_2$ (XXc)
 |
 $R^{\gamma'}$

dans lesquelles $R^{\gamma}$ a la définition donnée ci-dessus en 1°) et $R^{\gamma'}$ est un substituant défini ci-dessus en 2°), en présence d'un alcoolate alcalin, par exemple l'éthylate ou le méthylate de sodium, ou le t.butylate de potassium, par application de la méthode décrite par M. PESSON et M. ANTOINE, Bull. Soc. Chim. France 1590 (1970).

Il n'est pas absolument nécessaire de purifier le produit obtenu (ni de libérer les radicaux protégés) pour le mettre en oeuvre pour la préparation des produits de formule générale (XII).

La thiosemicarbazide de formule générale (XXa), (XXb) ou (XXc) peut être préparée selon l'une des méthodes décrites par K.A. JENSEN et coll., Acta Chem. Scand., *22*, 1 (1968), ou par application de la méthode décrite par Y. KAZAKOV et J.Y. POTOVSKII, Doklady Acad. Nauk. SSSR, *134*, 824 (1960), étant entendu que, lorsque $R^{\gamma}$ contient un radical amino, ce dernier est protégé.

La protection du radical amino et l'élimination du radical protecteur s'effectuent selon les méthodes habituelles qui n'altèrent pas le reste de la molécule. On utilise notamment le groupement t.butoxycarbonyle, qui peut être éliminé par hydrolyse acide.

— Lorsque R est un radical alcoyl-1triazol-1,3,4 yle-5: par application de l'une des méthodes décrites par M. PESSON et M. ANTOINE, Bull. Soc. Chim. France 1590 (1970):

— Lorsque R est un radical dioxo-5,6 tétrahydro-1,4,5,6 triazine-1,2,4 yle-3 substitué en position -4 par acyloxyalcoyle (éventuellement substitué): par acylation de la dioxo-5,6 hydroxyalcoyl-4 mercapto-3 tétrahydro-1,4,5,6 triazine-1,2,4 dont le radical mercapto a été préalablement protégé [par exemple selon C.G. KRUSE et coll., Tet. Lett. 1725 (1976)], par toute méthode connue pour acyler un alcool sans toucher au reste de la molécule, puis libération du groupement mercapto en milieu acide.

— Lorsque R est un radical alcoyl-2 dioxo-5,6 tétrahydro-1,2,5,6 triazine-1,2,4 yle-3, alcoyl-1 triazol-1,2,4 yle-5 ou alcoyl-1 alcoyloxycarbonyl-3 triazol-1,2,4 yle-5: selon la méthode décrite par M. PESSON et M. ANTOINE, C.R. Acad. Sci., Série C, *267*(25),1726 (1968).

— Lorsque R est un radical alcoyl-2 dihydro-2,5 oxo-5 triazine-1,2,4 yle-3 substitué en position -6 par un radical alcoyle ou alcoyloxy: selon la méthode décrite dans J. Antibiotics, *33*, 783 (1980).

— Lorsque R est un radical amino-1 dihydro-1,2 oxo-2 pyrimidinyle-4: selon la méthode décrite dans la demande de brevet européen EP 00005.

— Lorsque R est un radical thiadiazol-1,3,4 yle-5 éventuellement substitué par alcoyle: selon les méthodes décrites dans le brevet belge 830 821.

— Lorsque R est un radical thiadiazol-1,3,4 yle-5 substitué par dialcoylaminoalcoyle: selon la méthode décrite dans la demande de brevet allemand 2 446 254.

— Lorsque R est un radical thiadiazol-1,3,4 yle-5 substitué par un radical acylaminoalcoyle: selon la méthode décrite dans la demande de brevet japonais 76 80857.

— Lorsque R est un radical tétrazolyle-5 éventuellement substitué en position -1 par alcoyle ou hydroxyalcoyle: selon les méthodes décrites dans le brevet belge 830 821.

— Lorsque R est un radical tétrazolyle-5 substitué en position -1 par un radical dialcoylaminoalcoyle: par application de la méthode décrite dans la demande de brevet allemand 2 738 711.

— Lorsque R est un radical tétrazolyle-5 substitué par un radical acylaminoalcoyle: selon la méthode décrite dans le brevet US 4 117 123.

— Lorsque R est un radical tétrazolyle-5 substitué en position -1 par un radical dihydroxy-2,3 propyle: selon la méthode décrite dans le brevet US 4 064 242.

— Lorsque R est un radical tétrazolyle-5 substitué en position -1 par un radical dihydroxy-1,3 propyle-2: par addition d'azoture de sodium sur un isothiocyanate de diméthyl-2,2 dioxolanne-1,3 yle-5 (suivie éventuellement de la libération des groupements hydroxy).

— Lorsque R est un radical tétrazolyle-5 substitué en position -1 par un radical de formule générale (XIXa): par acition d'azoture de sodium sur l'isothiocyanate correspondant, par analogie avec la méthode décrite par R.E. ORTH, J. Pharm. Sci. *52* (9), 909 (1963).

II/ - Les thiovinyl-3 oxacéphalosporines de formule générale (XII) peuvent être également préparées en opérant de la manière suivante:

A) - On fait agir un thiol de formule générale (XVIII) dont le radical R est éventuellement protégé, ou un de ses sels alcalins ou alcalino-terreux, sur un dérivé d'oxacéphalosporine, ou le cas échéant sur un mélange d'isomères bicyclooctène-2 ou -3 d'un dérivé de formule générale (XIV) dans laquelle $R'_1$, $R_{14}$, $R_4$ et R'' sont définis comme en $b_1$), pour obtenir un dérivé d'oxacéphalosporine de formule générale:

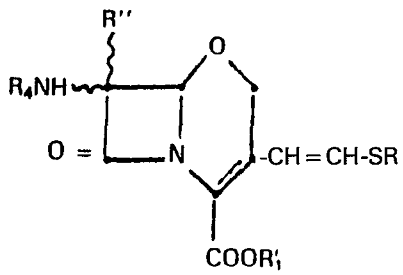

(XXI)

dans laquelle R, $R'_1$, $R_4$ et R'' sont définis comme ci-dessus.

La réaction s'effectue généralement dans les conditions décrites précédemment pour l'obtention d'une thiovinyl-3 oxacéphalosporine de formule générale (XII) à partir d'un thiol de formule générale (XVIII) et d'un produit de formule générale (XIV).

B) - On opère alors selon l'un ou l'autre des schémas suivants:

$B_1$ - On élimine le radical $R_4$ (ou éventuellement le radical $R_4$ et les autres radicaux protecteurs, successivement ou simultanément) d'un produit de formule générale (XXI), pour préparer une amino-7 oxacéphalosporine de formule générale:

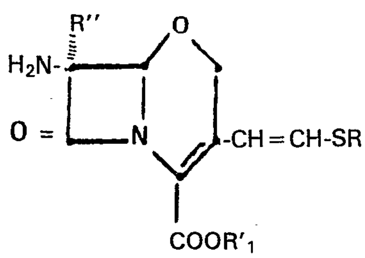

(XXIIa)

ou

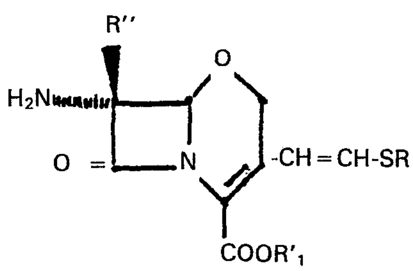

(XXIIb)

dans laquelle $R'_1$ représente un atome d'hydrogène ou un radical protecteur tel que défini précédemment, R est défini comme précédemment et R'' représente un atome d'hydrogène ou un radical méthoxy en 7$\alpha$, pour la formule générale (XXIIa) ou R'' représente un atome d'hydrogène en position 7$\beta$, pour la formule générale (XXIIb), puis lorsque R'' représente un atome d'hydrogène en 7$\beta$, ou lorsque l'on veut obtenir un produit de formule générale (XXIIa) dans laquelle R'' est méthoxy, on transforme l'oxacéphalosporine de formule générale (XXIIa) [dans laquelle R'' est hydrogène], ou (XXIIb), en le dérivé méthoxylé correspondant.

L'élimination du radical $R_4$ s'effectue par exemple dans les conditions décrites précédemment pour l'obtention d'un produit de formule générale (XVII) à partir d'un produit de formule générale (XIV).

La méthoxylation s'effectue dans les conditions décrites dans le brevet japonais 77 125 185 ou dans les brevets belges 871 213 et 863 998, ou selon les méthodes décrites par T. KOBAYASHI et coll., Chem. Pharm. Bull. *27*(11) 2718 (1979) ou par H. YANAGISAWA et coll., Tet. Lett., *31*, 2705 (1975).

$B_2$ - Ou bien, lorsque l'on veut obtenir un produit de formule générale (XXIIa) dans laquelle R'' est méthoxy, on méthoxyle une oxacéphalosporine de formule générale(XXI) dans laquelle R'' représente un atome d'hydrogène en 7$\beta$ et $R_4$ représente un radical facilement éliminable de formule générale (IVa), (IVe), (IVf), (IVg) ou un radical bis(nitro-4 benzyl)-phosphoryle, en opérant dans les conditions décrites ci-dessus en $B_1$, puis on élimine le radical $R_4$ (ou éventuellement le radical $R_4$ et les autres radicaux protecteurs).

La libération du radical amino s'effectue comme décrit précédemment pour la préparation de l'oxacéphalosporine de formule générale (XVII).

C) - On fait alors agir un acide de formule générale:

R'-OH (XXIII)

dans laquelle R' est défini comme précédemment (étant entendu que, lorsque R' est un radical de formule générale (II), la fonction amine de ce radical est protégée) ou un dérivé réactif de cet acide, sur une amino-7 oxacéphalosporine de formule générale (XXIIa), puis on élimine les groupements protecteurs.

— Lorsque R' représente un radical de formule générale (II), l'acide de formule générale (XXIII), sous forme syn ou anti ou leurs mélanges, conduit respectivement aux produits de formule générale(I) de forme syn ou anti ou leurs mélanges.

Il est entendu que l'oxime est protégée lorsque $R_3$ représente un atome d'hydrogène.

Lorsque $R_3$ contient un radical carboxy, celui-ci est également protégé.

— Lorsque R' représente un radical $\alpha$-carboxy-arylacétyle la protection du groupement carboxy n'est pas obligatoire; il peut donc être libre ou protégé.

Il en est de même pour le radical hydroxy lorsque le groupement aryle représente p.hydroxyphényle.

— Lorsque R contient un substituant amino ou alcoylamino, ce groupement est protégé, et lorsqu'il contient un ou plusieurs substituants hydroxy, ceux-ci sont libres ou de préférence protégés.

Il est entendu que les groupements amino, alcoylamino, carboxy et hydroxy qui existent dans certains radicaux sont (ou peuvent être) protégés par tous groupements protecteurs habituellement utilisés pour la protection des amines, des acides carboxyliques, des alcools ou des oximes et dont la mise en oeuvre n'altère pas le reste de la molécule.

On utilise par exemple des groupements protecteurs tels que cités précédemment.

— Lorsque l'on veut obtenir un produit de formule générale (XII) dans laquelle R contient un radical formylalcoyle, ce radical peut être éventuellement protégé à l'état d'acétal, sous forme d'un radical de formule générale (XIXa) ou (XIXb). Ce radical peut être éliminé dans les conditions décrites précédemment.

a) Lorsque l'on utilise le produit de formule générale (XXIII) sous forme acide, on effectue généralement la condensation de ce produit (dont le cas échéant les fonctions amine et/ou oxime ont été préalablement protégées) sur l'amino-7 oxacéphalosporine de formule générale (XXIIa) dans laquelle, R étant défini comme précédemment, $R'_1$ représente un radical protecteur facilement éliminable, en opérant dans un solvant organique tel que le diméthylformamide, l'acétonitrile, le tétrahydrofuranne, le dichlorométhane ou le chloroforme, en présence d'un agent de condensation tel qu'un carbodiimide (par exemple le dicyclohexylcarbodiimide), le NN'-carbodiimidazole ou l'éthoxy-2 éthoxycarbonyl-1 dihydro-1,2 quinoléine, à une température comprise entre —20 et 40°C puis on élimine les groupements protecteurs présents dans la molécule. Eventuellement on opère en présence d'une quantité catalytique de N,N-diméthylamino-4 pyridine.

b) Lorsque l'on utilise un dérivé réactif de l'acide de formule générale (XXIII), il est possible de mettre en oeuvre l'anhydride, un anhydride mixte ou un ester réactif de formule générale:

R'-OZ (XXIV)

dans laquelle R' est défini comme ci-dessus et Z représente un radical succinimido, benzotroazolyle-1, nitro-4 phényle, dinitro-2,4 phényle, pentachlorophényle ou phthalimido. Lorsque R' est un radical de formule générale (II), la fonction amine de tels dérivés est préalablement protégée (par exemple comme décrit précédemment). Les conditions de protection des différents substituants sont telles que décrites précédemment.

Il est également possible de mettre en oeuvre un dérivé réactif tel qu'un halogénure d'acide. Dans ce dernier cas, lorsque R' est un radical de formule générale (II) on peut, par exemple, faire réagir le chlorhydrate du chlorure d'acide sur l'amino-7 oxacéphalosporine de formule générale (XXIIa). Lorsque R' est un radical α-carboxy (p.hydroxyphényl) acétyle, le groupement hydroxy peut être libre ou protégé.

Lorsque l'on met en oeuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple acétone), ou dans un mélange de tels solvants en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine, (par exemple triéthylamine), ou dans un milieu hydroorganique en présence d'un agent alcalin de condensation tel que le bicarbonate de sodium, et l'on opère à une température comprise entre —40 et +40°C, puis on remplace éventuellement les groupements protecteurs par des atomes d'hydrogène.

Lorsque l'on met en oeuvre un ester réactif de formule générale (XXIV), on opère généralement en présence d'une trialcoylamine (par exemple triéthylamine) dans un solvant organique tel que le diméthylformamide à une température comprise entre 0 et 40°C, puis on remplace les groupements protecteurs par des atomes d'hydrogène.

Les isomères de produits de formule générale (I), (V), (VIII), (IXa), (XII), (XIV), (XIVa), (XVII), (XXI), (XXIIa) et (XXIIb) peuvent être séparés par chromatographie ou cristallisation.

Les nouveaux dérivés de l'oxacéphalospirine de formule générale (XII) et leurs sels pharmaceutiquement acceptables présentent des propriétés antibactériennes particulièrement intéressantes. Ils manifestent une activité remarquable in vitro et in vivo sur les germes Gram-positifs et Gram-négatifs.

In nitro, les produits de formule générale (XII) se sont montrés actifs à une concentration comprise entre 1 et 15 $\mu g/cm^3$ sur des souches de staphylocoques sensibles à la pénicilline G (Staphylococcus aureus Smith), à une concentration comprise entre 0,01 et 1 $\mu g/cm^3$ sur Escherichia coli souche NIHJ.

In vitro, les produits de formule générale (XII) se sont montrés actifs sur les infections expérimentales de la souris à Staphylococcus aureus Smith (sensible à la pénicilline G) à une dose comprise entre 0,5 et 15 mg/kg par jour par voie sous-cutanée, et à Escherichia coli souche NIHJ à des doses comprises entre 0,01 et 10 mg/kg par jour par voie sous-cutanée.

Par ailleurs, la $DL_{50}$ des produits de formule générale (XII) est comprise entre 1,5 g/kg et des doses supérieures à 2,5 g/kg par voie sous-cutanée chez la souris.

D'un intérêt particulier sont les oxacéphalosporines de formule générale (I) de forme bicyclooctène-2 dans laquelle $R_1$ représente un radical benzhydrile ou nitrobenzyle, $R_4$ représente un radical de fo-mule générale (II), ou un radical facilement éliminable [choisi parmi trityle ou les radicaux de formule générale (IVa) et (IVd)] et R'' représente un atome d'hydrogène ou un radical méthoxy en 7α.

Et parmi ces produits, plus spécialement, les produits de formule générale (I) de forme bicyclooctène-2 dans laquelle $R_1$ représente un radical benzhydryle ou p. nitrobenzyle, $R_4$ représente un radical de formule générale (II) [dans laquelle $R_2$ représente un radical protecteur d'amino et $R_3$ représente un radical alcoyle] ou un radical facilement éèiminable choisi parmi trityle ou les radicaux de formule générale (IVa) [dans laquelle $R_5$ représente un radical de formule générale (IVb) dans laquelle Y représente un atome d'oxygène ou de formule générale (IVc)], ou les radicaux de formule générale (IVd) dans laquelle $R_6$ représente un radical alcoyle, et R'' représente un atome d'hydrogène ou un radical méthoxy en 7α.

Et notamment:

— le benzhydryloxycarbonyl-2 (oxo-2 éthyl)-3 oxo-8 tritylamino-7 oxa-5 aza-1 bicyclo[4.2.0] octène-2

— le benzhydryloxycarbonyl-2 (oxo-2 éthyl)-3 oxo-8 phénylacétamido-7 oxa-5 aza-1 bicyclo[4.2.-0] octène-2

— le benzhydryloxycarbonyl-2 méthoxy-7α (oxo-2 éthyl)-3 oxo-8 phénoxyacétamido-7β oxa-5 aza-1 bicyclo[4.2.0] octène-2 et

— le benzhydryloxycarbonyl-2 [méthoxyimino-2 (tritylamino-2 thiazolyl-4)-2 acétamido]-7 (oxo-2 éthyl)-3 oxo-8 oxa-5 aza-1 bicyclo[4.2.0] octène-2.

Les exemples suivants, donnés à titre non limitatif, montrent comment l'invention peut être mise en pratique.

Dans ces exemples les produits sont cités selon la nomenclature des Chemical Abstracts. Il est entendu qu'en l'absence de mention particulière les dérivés d'oxacéphalosporine qui sont cités présentent la stéréochimie donnée par la formula générale partielle:

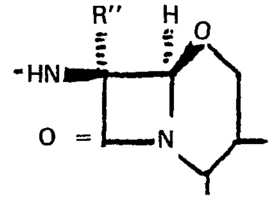

(XXV)

dans laquelle R'' est en position 7$\alpha$.

*Exemple 1*

Une solution de 3,0 g de benzhydryloxycarbonyl-2 (diméthylamino-2 vinyl)-3 oxo-8 tritylamino-7 oxa-5 aza-1 bicyclo[4.2.0] octène-2, forme E, dans 100 $cm^3$ d'acétate d'éthyle est agitée vigoureusement pendant 1 heure 30 à 20°C en présence de 45 $cm^3$ d'une solution d'acide chlorhydrique 1N. Le mélange est décanté et la phase organique est lavée par 50 $cm^3$ d'une solution à 5% de bicarbonate de sodium puis par 50 $cm^3$ d'une solution demi-saturée de chlorure de sodium. La phase acétate d'éthyle est séchée sur sulfate de sodium, filtrée et concentrée à sec à 30°C sous pression réduite (20 mm de mercure; 2,7 kPa). On obtient 2,52 g d'un produit brut constitué principalement de benzhydryloxycarbonyl-2 oxo-8 (oxo-2 éthyl)-3 tritylamino-7 oxa-5 aza-1 bicyclo[4.2.0] octène-2, sous la forme d'une meringue marron clair.

Spectre infra-rouge (KBr), bandes caractéristiques ($cm^{-1}$): 1790, 1720, 1600, 1495, 1450, 1220, 750, 700.

Spectre de RMN du proton (350 MHz, $CDCl_3$, $\delta$ en ppm, J en Hz): 3,37 et 3,50 (2d, J = 16, 2H, $-C\underline{H}_2{<}^{O}_{H}$); 3,81 (d, J = 3,5, 1H, H en 6); 3,92 et 4,12 (2d, J = 18, 2H, $-CH_2-O-$); 4,35 (dd, J = 3,5 et 9, 1H, H en 7); 6,80 [s, 1H, $-COO-CH(C_6H_5)_2$]; 9,49 (s, 1H, $-C{<}^{O}_{H}$).

Le benzhydryloxycarbonyl-2 (diméthylamino-2 vinyl)-3 oxo-8 tritylamino-7 oxa-5 aza-1 bicyclo-[4.2.0] octène-2, forme E peut être obtenu de la manière suivante:

On chauffe à 80°C sous azote, une solution de 4,25 g de benzhydryloxycarbonyl-2 méthyl-3 oxo-8 tritylamino-7 oxa-5 aza-1 bicyclo[4.2.0] octène-2 dans 20 $cm^3$ de diméthylformamide. On ajoute, goutte à goutte en 7 minutes, dans la solution maintenue sous agitation à 80°C, 1,55 $cm^3$ de t.butoxy bis-diméthylaminométhane. L'addition terminée, le mélange est conservé sous agitation à 80°C pendant 17 minutes. La solution est diluée par 150 $cm^3$ d'acétate d'éthyle, la phase organique est lavée par trois fois 60 $cm^3$ d'eau distillée et 60 $cm^3$ d'une solution demi-saturée de chlorure de sodium, séchée sur sulfate de sodium, filtrée et concentrée à sec à 30°C sous pression réduite (20 mm de mercure; 2,7 kPa). Le résidu est trituré dans 150 $cm^3$ d'éther éthylique, la suspension obtenue est filtrée et le filtrat est concentré à sec à 30°C sous pression réduite (20 mm de mercure; 2,7 kPa). On obtient 3,14 g d'un produit brut constitué principalement de benzhydryloxycarbonyl-2 (diméthylamino-2 vinyl)-3 oxo-8 tritylamino-7 oxa-5 aza-1 bicyclo[4.2.0] octéne-2, forme E utilisable sans purification supplémentaire.

Spectre infra-rouge (KBr), bandes caractéristiques ($cm^{-1}$): 1780, 1660, 1615, 1490, 1450, 745, 700.

Spectre de RMN du proton (350 MHz, $CDCl_3$, $\delta$ en ppm, en Hz): 2,77 [s, 6H, $-N(CH_3)_2$]; 3,71 (d, J = 3,5, 1H, H en 6); 4,12 et 4,53 (2d, J = 17, 2H, $-CH_2-O-$); 4,26 (mf, 1H, H en 7); 6,24 et 6,40 (2d, J = 13, 2H, -CH=CH-); 6,81 [s, 1H, $-COO-CH(C_6H_5)_2$].

7,74 g de benzhydryloxycarbonyl-2 méthyl-3 oxo-8 tritylamino-7 oxa-5 aza-1 bicyclo[4.2.0] octéne-2 sont préparés selon un schéma de synthese décrit dans le brevet américain 4 108 992 dans lequel on remplace le glyoxylate de t-butyle par le glyoxylate de benzhydryle préparé selon le brevet français 1 495 047.

L'oxacéphalosporine attendue est obtenue sous la forme d'un solide blanc à partir de 13,2 g d'oxo-2 (propyne-2 yloxy)-4 tritylamino-3 azétidine.

Spectre infra-rouge (KBr), bandes caractéristiques ($cm^{-1}$): 3340, 1780, 1715, 1620, 1595, 1585, 1490, 1450, 1220, 745, 700.

Spectre de RMN du proton (350 MHz, $CDCl_3$, $\delta$ en ppm, J en Hz): 1,90 (s, 3H, $-CH_3$); 3,75 (d, J = 3,5, 1H, H en 6); 3,87 et 4,08 (2d, J = 18, 2H, $-CH_2-O-$); 4,30 (d, J = 3,5, 1H, H en 7); 6,85 [s, 1H, $-COO-C\underline{H}(C_6H_5)_2$]; 7,15 à 7,4 (Mt, 26H, aromatiques et $-\underline{H}N-C(C_6H_5)_3$.

*Exemple 2*

Une solution de 1,05 g de benzhydryloxycarbonyl-2 (diméthylamino-2 vinyl)-3 ]méthoxyimino-2 (tritylamino-2 thiazolyl-4)-2 acétamido]-7 oxo-8 oxa-5 aza-1 bicyclo[4.2.0] octéne-2, isomère syn, forme E dans 10 $cm^3$ d'acétate d'éthyle est agitée vigoureusement pendant 1 heure à 20°C en présence de 5 $cm^3$ d'une solution 1N d'acide chlorhydrique. Le mélange est décanté et la phase organique est lavée par 10 $cm^3$ d'une solution saturée de bicarbonate de sodium, 10 $cm^3$ d'eau distillée et 10 $cm^3$ d'une solution saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 1,00 g de benzhydryloxycarbonyl-2 [méthoxyimino-2 (tritylamino-2 thiazolyl-4)-2 acétamido]-7 oxo-8 (oxo-2 éthyl)-3 oxa-5 aza-1 bicyclo-[4.2.0] octène-2, isomère syn sous la forme d'une meringue jaune.

Spectre infra-rouge (KBr), bandes caractéristiques ($cm^{-1}$): 3400, 1795, 1725, 1685, 1525, 1495, 1450, 1035, 755, 700.

Spectre de masse: pic moléculaire = 817.

Spectre de RMN du proton (250 MHz, $CDCl_3$, $\delta$ en ppm, J en Hz): 3,52 (D, J = 16,25, 1H, $-C\underline{H}_2CHO$); 3,62 (D, J = 16,25, 1H, $-C\underline{H}_2-CHO$); 4,07 (s, 3H, $=NOCH_3$); 4,35 (s, 2H, $-CH_2O-$); 5,17 (D, J = 3,75, 1H, -H en 6); 5,82 (DD, J = 10 et 3,75, 1H, -H en 7);

6,76 (D, J = 10, 1H, -CONH-); 6,80 (s, 1H, -H du thiazole); 6,85 [s, 1H, -COOCH$(C_6H_5)_2$]; 7,13 [Mf, 1H, $(C_6H_5)_3$ CNH-]; 7,20 à 7,55 (Mf, 25H, aromatiques); 9,60 (s, 1H, -CHO).

Le benzhydryloxycarbonyl-2 (diméthylamino-2 vinyl)-3 [méthoxyimino-2 (tritylamino-2 thiazolyl-4)-2 acétamido]-7 oxo-8 oxa-5 aza-1 bicyclo[4.2.0] octène-2, isomère syn, forme E peut être obtenu de la manière suivante:

On chauffe à 80°C sous azote, une solution de 0,79 g de benzhydryloxycarbonyl-2 [méthoxyimino-2 (tritylamino-2 thiazolyl-4)-2 acétamido]-7 méthyl-3 oxo-8 oxa-5 aza-1 bicyclo[4.2.0] octène-2, isomère syn, dans 5 $cm^3$ de diméthylformamide. On ajoute goutte à goutte en 6 minutes, dans la solution maintenue sous agitation à 80°C, 0,2 $cm^3$ de t.butoxy bis-diméthylaminométhane. L'addition terminée, le mélange est conservé sous agitation à 80°C pendant 20 minutes. La solution est diluée par 25 $cm^3$ d'acétate d'éthyle, la phase organique est lavée par trois fois 25 $cm^3$ d'eau distillée et 25 $cm^3$ d'une solution saturée de chlorure de sodium, séchée sur sulfate de magnésium, filtrée et concentrée à sec à 40°C sous pression réduite (20 mm de mercure; 2,7 kPa). On obtient 0,71 g d'un produit brut constitué principalement de benzhydryloxycarbonyl-2 (diméthylamino-2 vinyl)-3 [méthoxyimino-2 (tritylamino-2 thiazolyl-4)-2 acétamido]-7 oxo-8 oxa-5 aza-1 bicyclo[4.2.0] octéne-2, isomère syn, forme E.

Un échantillon (0,4 g) est purifié par chromatographie sur une colonne de silice (0,04-0,06) (diamètre de la colonne: 2,2 cm; hauteur: 20 cm) en éluat sous 60 kPa avec un mélange de cyclohexane et d'acétate d'éthyle (40-60 envolumes) et en recueillant des fractions de 40 $cm^3$. Les fractions 9 à 15 sont rassemblées et concentrées à sec sous pression réduite (30 mm de mercure; 4 kPa) à 40°C. On obtient 0,020 g d'une poudre jaune du produit pur.

Spectre de masse: pic moléculaire = 844.

Spectre de RMN du proton (350 MHz, $CDCl_3$, $\delta$ en ppm, J en Hz): 2,86 [s, 6H, -N$(CH_3)_2$]; 4,07 (s, 3H, =$NOCH_3$); 4,61 et 4,76 (2D, J = 18, 2H, -$CH_2$O-); 5,10 (D, J = 3,5, 1H, H en 6); 5,69 (DD, J = 3,5 et 9, 1H, H en 7); 6,36 et 6,54 (2D, J = 14, 2H, -CH=CH-); 6,67 (D, J = 9, 1H, -CONH-); 6,82 (D, 1H, H thiazole); 6,86 [s, 1H, -$CO_2$CH$(C_6H_5)_2$]; 7,01 [s, 1H, -NH C$(C_6H_5)_3$]; 7,15 à 7,65 (m, 25H).

Spectre infra-rouge (KBr), bandes caractéristiques ($cm^{-1}$: 1780, 1685, 1615, 1525, 1490, 1445, 1120, 1030, 740, 695.

Le benzhydryloxycarbonyl-2 [méthoxyimino-2 (tritylamino-2 thiazolyl-4)-2 acétamido]-7 méthyl-3 oxo-8 oxa-5 aza-1 bicyclo[4.2.0] octéne-2, isomère syn, peut être prépare de la manière suivante:

Une solution de 35 g de tosylate d'amino-7 benzhydryloxycarbonyl-2 méthyl-3 oxo-8 oxa-5 aza-1 bicyclo[4.2.0] octéne-2 dans 50 $cm^3$ d'acétate d'éthyle est lavée par 15 $cm^3$ d'une solution 1N de bicarbonate de sodium et 50 $cm^3$ d'eau distillée puis séchée sur sulfate de magnésium. Après filtration, le solvant est évaporé sous pression réduite (20 mm de mercure; 2,7 kPa) à 40°C. On obtient 2,33 g d'amino-7 benzhydryloxycarbonyl-2 méthyl-3 oxo-8 oxa-5 aza-1 bicyclo[4.2.0] octéne-2 sous forme d'une meringue jaune pâle.

Spectre de masse: pic moléculaire = 364.

Spectre infra-rouge (KBr), bandes caractéristiques ($cm^{-1}$: 3400, 3340, 1780, 1720, 1640, 1495, 1445, 1230, 1110, 1060, 745, 700.

Spectre de RMN du proton (350 MHz, $CDCl_3$, $\delta$ en ppm, J en Hz): 2,03 (s, 3H, -$CH_3$); 4,32 (s, 2H, -$CH_2$O-); 4,47 (D, J = 3,5, 1H, -H en 7); 4,92 (D, J = 3,5, 1H, -H en 6); 6,90 [s, 1H, -$CO_2$CH $(C_6H_5)_2$]; 7,15 à 7,45 (m, 10H aromatiques).

A une solution de 2,3 g d'amino-7 benzhydryloxycarbonyl-2 méthyl-3 oxo-8 oxa-5 aza-1 bicyclo-[4.2.0] octéne-2, de 2,79 g d'acide méthoxyimino-2 (tritylamino-2 thiazolyl-4)-2 acétique, isomère syn, dans 50 $cm^3$ de chlorure de méthylène, on ajoute 1,56 g de N,N'-dicyclohexylcarbodiimide et 0,001 g de N,N-diméthylamino-4 pyridine en solution dans 40 $cm^3$ de chlorure de méthylène pendant 15 minutes. Le mélange est maintenu sous agitation à 0°C pendant 2 heures. Il est alors transféré dans une ampoule à décanter, puis lavé successivement par 50 $cm^3$ d'une solution 0,1 N d'acide chlorhydrique, 50 $cm^3$ d'eau distillée, 50 $cm^3$ d'une solution demi-saturée de bicarbonate de sodium puis 50 $cm^3$ d'eau distillée. La phase chlorométhylénique est séchée sur sulfate de magnésium anhydre. Le séchant filtré, la solution est concentrée à sec sous pression réduite (100 mm de mercure; 13,5 kPa). Le résidu (5,6 g) est purifié par chromatographie sur une colonne de silice (0,04-0,06) (diamètre de la colonne: 5,6 cm; hauteur: 26 cm) en éluant sous 50 kPa avec un mélange de cyclohexane et d'acétate d'éthyle (50-50 en volumes) et en recueillant des fractions de 120 $cm^3$. Les fractions 8 à 20 sont rassemblées et concentrées à sec sous pression réduite (30 mm de mercure; 4 kPa) à 40°C. On obtient 4,02 g d'une meringue beige du benzhydryloxycarbonyl-2 [méthoxyimino-2 (tritylamino-2 thiazolyl-4)-2 acétamido]-7 méthyl-3 oxo-8 oxa-5 aza-1 bicyclo[4.2.0] octène-2, isomère syn, pur.

Spectre de masse: pic moléculaire = 789.

Spectre infra-rouge (KBr), bandes caractéristiques ($cm^{-1}$: 2820, 1790, 1745, 1580, 1530, 1495, 1450, 1165, 1040, 750, 700.

Spectre de RMN du proton (350 MHz, $CDCl_3$, $\delta$ en ppm, J en Hz): 2,04 (s, 3H, -$CH_3$); 4,07 (s, 3H, =N-$OCH_3$); 4,32 (s, 2H, -$CH_2$O-); 5,12 (D, J = 3,5, 1H, -H en 6); 5,75 (DD, J = 3,5 et 9, 1H, -H en 7); 6,68 (D, J = 9, 1H, -CONH-); 6,79 (s, 1H, H thiazole); 6,89 [s, 1H, -$CO_2$CH $(C_6H_5(_2]$; 7,00 [s large, 1H, -NH C$(C_6H_5)_3$]; 7,15 à 7,55 (m, 25H aromatiques).

Le tosylate d'amino-7 benzhydryloxycarbonyl-2 méthyl-3 oxo-8 oxa-5 aza-1 bicyclo[4.2.0] octène-2 peut être obtenu de la manière suivante:

On agite à 40°C pendant 30 minutes une solution de 6,07 g de benzhydryloxycarbonyl-2 méthyl-3 oxo-8 tritylamino-7 oxa-5 aza-1 bicyclo[4.2.0] octène-2 et 1,9 g d'acide p.toluènesulfonique hydraté dans 100 $cm^3$ d'acétone. Le mélange est refroidi à 0°C et un solide blanc précipite. On essore et on lave le gâteau par deux fois 5 $cm^3$ d'acétone. On obtient ainsi 3,17 g de tosylate d'amino-7 benzhydryloxycarbonyl-2 méthyl-3 oxo-8 oxa-5 aza-1 bicyclo-[4.2.0] octène-2.

Le filtrat acétonique est concentré à sec sous pression réduite (20 mm de mercure; 2,7 kPa). Le résidu

est trituré dans deux fois 100 $cm^3$ d'éther éthylique. L'éther est décanté et le solide obtenu est repris dans 20$cm^3$ d'acétate d'éthyle. On filtre et on obtient une deuxième fraction de 0,72 g de tosylate d'amino-7 benzhydryloxycarbonyl-2 méthyl-3 oxo-8 oxa-5 aza-1 bicyclo[4.2.0] octène-2.

Spectre infra-rouge ($CHBr_3$), bandes caractéristiques ($cm^{-1}$): 3300, 2400, 1800, 1720, 1500, 1455, 1225, 820, 760, 745, 570.

Spectre de RMN du proton (350 MHz, $CDCl_3$, $\delta$ en ppm, J en Hz): 1,73 (s, 3H, $-CH_3$); 2,15 [s, 3H, $-CH_3$ (APTS)]; 398 (D, J = 19, 1H, $-CH_2O-$); 4,11 (D, J = 19, 1H, $-CH_2O-$); 4,78 (D, J = 2,5, 1H, -H en 7); 4,86 (D, J = 2,5, 1H, -H en 6); 6,87 [s, 1H, $-COOC\underline{H}$ $(C_6H_5)_2$]; 6,96 (D, J = 7,5, 1H, -H en ortho du méthyle, APTS); 7,10 à 7,60 (Mt, 10H aromatiques); 7,74 (D, J = 7,5, 1H, -H en ortho du $SO_3H$, APTS); 8,60 (Mf, 3H, $NH_3^+$).

*Exemple 3*

Une solution de 0,16 g du benzhydryloxycarbonyl-2 t.butoxycarbonylamino-7 (diméthylamino-2 vinyl)-3 oxo-8 oxa-5 aza-1 bicyclo[4.2.0] octène-2, forme E (obtenu comme décrit ci-après) dans 5 $cm^3$ d'acétate d'éthyle est agitée vigoureusement pendant 30 minutes à 20°C en présence de 3 $cm^3$ d'une solution d'acide chlorhydrique 1N. Le mélange est décanté et la phase organique est lavée par 5 $cm^3$ d'une solution à 5% de bicarbonate de sodium, 5 $cm^3$ d'eau distillée, puis 5 $cm^3$ d'une solution demi-saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 0,11 g de benzhydryloxycarbonyl-2 t.butoxycarbonylamino-7 (oxo-2 éthyl)-3 oxo-8 oxa-5 aza-1 bicyclo[4.2.0] octène-2, sous la forme d'une meringue marron clair.

Spectre de masse: pic moléculaire = 492.

Spectre infra-rouge (KBr), bandes caractéristiques ($cm^{-1}$): 1790, 1720, 1515, 1500 + 1455, 1390, 1370, 1165, 750, 700.

Spectre de RMN du proton (350 MHz, $CDCl_3$, $\delta$ en ppm, J en Hz): 1,47 [s, 9H, $-C(CH_3)_3$]; 3,52 (D, J = 16, 1H, $-C\underline{H_2}-CHO$); 3,62 (D, J = 16, 1H, $-C\underline{H_2}$--CHO); 4,36 (AB limite, J ≃ 17,5, 2H, $-CH_2-O-$); 5,10 (D, J = 3,5, 1H, -H en 6); 5,27 (D, J = 10,5, 1H, -CONH-); 5,47 (DD, J = 10,5 et 3,5, 1H, -H en 7); 6,87 [s, 1H, -COOCH $(C_6H_5)_2$]; 7,20 à 7,60 (Mt, 10H aromatiques); 9,58 (s, 1H, -CHO).

Le benzhydryloxycarbonyl-2 t.butoxycarbonylamino-7 (diméthylamino-2 vinyl)-3 oxo-8 oxa-5 aza-1 bicyclo[4.2.0] octène-2, forme E peut être obtenu de la manière suivante:

On chauffe à 80°C, sous azote, une solution de 1,29 g de benzhydryloxycarbonyl-2 t.butoxycarbonylamino-7 méthyl-3 oxo-8 oxa-5 aza-1 bicyclo-[4.2.0] octène-2 dans 15 $cm^3$ de diméthylformamide. On ajoute goutte à goutte en 5 minutes, dans la solution maintenue sous agitation à 80°C, 0,7 $cm^3$ de t.butoxy bis-diméthylaminométhane. L'addition terminée, le mélange est maintenu sous agitation pendant 20 minutes. La solution est diluée par 100 $cm^3$ d'acétate d'éthyle, la phase organique est lavée par trois fois 50 $cm^3$ d'eau distillée et 50 $cm^3$ d'une solution saturée de chlorure de sodium, séchée sur sulfate de magnésium, filtrée et concentrée à sec à 30°C sous pression réduite (20 mm de mercure; 2,7 kPa). On obtient 1,10 g d'un produit brut constitué principalement de benzhydryloxycarbonyl-2 t.butoxycarbonylamino-7 (diméthylamino-2 vinyl)-3 oxo-8 oxa-5 aza-1 bicyclo[4.2.0] octène-2, forme E.

On chromatographie 1,04 g de ce produit sur une colonne de silice (0,04-0,06) diamètre de la colonne: 4,5 cm; hauteur: 25 cm) en éluant sous 50 kPa avec un mélange de cyclohexane et d'acétate d'éthyle (60-40 en volumes) et en recueillant des fractions de 50 $cm^3$. Les fractions 7 à 10 sont rassemblées et concentrées à sec sous pression réduite (30 mm de mercure; 4 kPa) à 40°C. On obtient 0,4 g d'une meringue jaune se présentant comme un mélange (44 à 56en moles) de benzhydryloxycarbonyl-2 t.butoxycarbonylamino-7 (diméthylamino-2 vinyl)-3 oxo-8 oxa-5 aza-1 bicyclo[4.2.0] octène-2, forme E et du produit (oxo-2 éthyl)-3 correspondant.

Caractéristiques physiques du benzhydryloxycarbonyl-2 t.butoxycarbonylamino-7 (diméthylamino-2 vinyl)-3 oxo-8 oxa-5 aza-1 bicyclo[4.2.0] octène-2, forme E.

Spectre de masse: pic moléculaire = 519.

Spectre infra-rouge (KBr), bandes caractéristiques ($cm^{-1}$: 3400, 2980, 2925, 2850, 1785, 1715, 1615, 1515, 1500, 1455, 1245, 1175, 1100, 1070, 750, 700.

Spectre de RMN du proton (350 MHz, $CDCl_3$, $\delta$ en ppm, J en Hz): 1,47 [s, 9H, $-C(CH_3)_3$]; 2,85 [s, 6H, $-N(CH_3)_2$]; 4,61 (D, J ≃ 16, 1H, $-CH_2O-$); 4,77 (D, J = 16, 1H, $-CH_2O-$); 5,10 (D, J = 3,5, 1H,-H en 6); 5,30 (D, J = 10,5, 1H, $-CON\underline{H}-$); 5,38 (DD, J = 10,5 et 3,5, 1H, -H en 7); 6,36 (D, J= 14, 1H, $-C\underline{H}=CH-$); 6,53 (D, J = 14, 1H, $-CH=C\underline{H}-$); 6,88 [s, 1H, $-COOC\underline{H}$ $(C_6H_5)_2$]; 7,14 à 7,70 (m, 10H aromatiques).

Le benzhydryloxycarbonyl-2 t.butoxycarbonylamino-7 méthyl-3 oxo-8 oxa-5 aza-1 bicyclo[4.2.0] octène-2 peut être obtenu de la manière suivante:

On agite pendant 4 heures à 20°C 1 g d'amino-7 benzhydryloxycarbonyl-2 méthyl-3 oxo-8 oxa-5 aza-1 bicyclo[4.2.0] octène-2, 0,45 g de bicarbonate de sodium, 0,65 g de pyrocarbonate de dit.butyle dans un mélange de 4 $cm^3$ d'eau distillée et 8 $cm^3$ de dioxanne. Le mélange est concentré sous pression réduite (20 mm de mercure; 2,7 kPa) jusqu'à un volume résiduel de 5 $cm^3$. Le résidu est dilué dans 10 $cm^3$ d'acétate d'éthyle, et la phase organique est lavée par 10 $cm^3$ d'eau distillée et séchée sur sulfate de magnésium. On filtre et le filtrat est évaporé sous pression réduite (20 mm de mercure; 2,7 kPa). On obtient 1,25 g de benzhydryloxycarbonyl-2 t.butoxycarbonylamino-7 méthyl-3 oxo-8 oxa-5 aza-1 bicyclo[4.2.0] octène-2 sous la forme d'une meringue jaune pâle.

Spectre de masse: pic moléculaire = 464.

Spectre infra-rouge (KBr), bandes caractéristiques ($cm^{-1}$: 3380, 1790, 1720, 1640, 1515, 1500, 1220, 1170, 750, 700.

Spectre de RMN du proton 350 MHz, $CDCl_3$, $\delta$ en ppm, J en Hz): 1,48 [s, 9H, $-C(CH_3)_3$]; 2,04 (s, 3H, $-CH_3$); 4,32 (s, 2H, $-CH_2O-$); 5,05 (D, J = 3,5, 1H, -H en 6); 5,26 (D, J = 10,5,1H, -CONH-); 5,43 (DD,J = 3,5 et 10,5, 1H, H en 7); 6,89 [s, 1H, $-CO_2C\underline{H}$

$(C_6H_5)_2]$; 7,23 à 7,60 (m, 10H aromatiques).

*Exemple 4*

Une solution de 0,02 g du benzhydryloxycarbonyl-2 (diméthylamino-2 vinyl)-3 oxo-8 phénylacétamido-7 oxa-5 aza-1 bicyclo[4.2.0] octène-2, forme E (obtenu dans les conditions décrites ci-après) dans 5 $cm^3$ d'acétate d'éthyle est agitée vigoureusement pendant 30 minutes à 20°C en présence de 3 $cm^3$ d'une solution d'acide chlorhydrique 1N. Le mélange est décante et la phase organique est lavée par 10 $cm^3$ d'eau distillée, 10 $cm^3$ d'une solution à 5% de bicarbonate de sodium, 10 $cm^3$ d'eau distillée et 10 $cm^3$ d'une solution demi-saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 0,02 g de benzhydryloxycarbonyl-2 (oxo-2 éthyl)-3 oxo-8 phénylacétamido-7 oxa-5 aza-1 bicyclo[4.2.0] octène-2, sous la forme d'une poudre jaune clair.

Spectre de masse: pic moléculaire = 510.

Spectre infra-rouge (KBr), bandes caractéristiques ($cm^{-1}$: 3420, 3370, 2930, 2860, 2740, 1792, 1720, 1685, 1670, 1650, 1530, 1500, 1460, 1230, 1110, 1070, 760, 750, 705.

Spectre de RMN du proton (350 MHz, $CDCl_3$, $\delta$ en ppm, J en Hz): 3,48 (D, J = 17,5, 1H, $-C\underline{H}_2CHO$); 3,54 (D, J = 17,5, 1H, $-C\underline{H}_2CHO$); 3,65 (s, 2H, $C_6H_6-CH_2-$); 4,22 (AB limite, J $\simeq$ 16, 2H, $-C\underline{H}_2-O-$); 5,05 (D, J = 3,5, 1H, -H en 6); 5,74 (DD, J = 10 et 3,5, 1H, -H en 7); 6,28 (D, J = 10, 1H, -CO-NH-); 6,84 [s, 1H, -COOCH $(C_6H_5)_2]$; 7,10 à 7,60 (Mt, 15H aromatiques); 9,53 (s, 1H, $-C\underline{H}O$).

Le benzhydryloxycarbonyl-2 (diméthylamino-2 vinyl)-3 oxo-8 phénylacétamido-7 oxa-5 aza-1 bicyclo[4.2.0] octène-2, forme E peut être obtenu de la manière suivante:

On chauffe à 80°C sous azote, une solution de 0,527 g de benzhydryloxycarbonyl-2 méthyl-3 oxo-8 phénylacétamido-7 oxa-5 aza-1 bicyclo-[4.2.0] octène-2 dans 5 $cm^3$ de diméthylformamide. On ajoute goutte à goutte en 4 minutes dans la solution maintenue sous agitation à 80°C, 0,25 $cm^3$ de t.butoxy bis-diméthylaminométhane. L'addition terminée, le mélange est conservé sous agitation à 80°C pendant 20 minutes. La solution est diluée par 25 $cm^3$ d'acétate d'éthyle, la phase organique est lavée par cinq fois 25 $cm^3$ d'eau distillée et 25 $cm^3$ d'une solution saturée de chlorure de sodium, séchée sur sulfate de magnésium, filtrée et concentrée à sec à 40°C sous pression réduite (20 mm de mercure; 2,7 kPa). On obtient 0,44 g d'un produit brut constitué principalement de benzhydryloxycarbonyl-2 (diméthylamino-2 vinyl)-3 oxo-8 phénylacétamido-7 oxa-5 aza-1 bicyclo[4.2.0] octène-2, forme E.

On chromatographie 0,4 g de ce produit sur une colonne de silice (0,04-0,06) (diamètre de la colonne: 3,9 cm; hauteur: 24 cm) en éluant sous 50 kPa avec un mélange de cyclohexane et d'acétate d'éthyle (40-60 en volumes) et en recueillant des fractions de 25 $cm^3$. Les fractions 25 à 30 sont rassemblées et concentrées à sec sous pression réduite (30 mm de mercure; 4 kPa) à 40°C. On obtient 0,030 g d'une meringue jaune se présentant comme un mélange (63-37 moles) de benzhydryloxycarbonyl-2 (diméthylamino-2 vinyl)-3 oxo-8 phénylacétamido-7 oxa-5 aza-1 bicyclo[4.2.0] octène-2 forme E et du dérivé (oxo-2 éthyl)-3 correspondant.

Caractéristiques physiques du benzhydryloxycarbonyl-2 (diméthylamino-2 vinyl)-3 oxo-8 phénylacétamido-7 oxa-5 aza-1 bicyclo[4.2.0] octène-2, forme E.

Spectre de masse: pic moléculaire = 537.

Spectre infra-rouge (KBr), bandes caractéristiques ($cm^{-1}$: 3430, 1785, 1725, 1690, 1620, 1540, 1500, 1460, 1440, 1220,1180, 1100, 760, 745, 705.

Spectre de RMN du proton (350 MHz, $CDCl_3$, $\delta$ en ppm, J en Hz): 2,82 [s, 6H, $-N(CH_3)_2]$; 3,65 (s, 2H, $C_6H_5-CH_2-$); 4,49 (D, J = 16, 1H, $-CH_2-O-$); 4,62 (D, J = 16, 1H, $-CH_2-O-$); 4,98 (D, J = 3,5, 1H, -H en 6); 5,62 (DD, J = 9 et 3,5, 1H, -H en 7); 6,28 (D, J = 9, 1H, -CONH-); 6,31 (D, J = 13,5, 1H, -CH=CH-); 6,45 (D, J = 13,5, 1H, -CH=CH-); 6,85 [s, 1H, -COOCH $(C_6H_5)_2]$; 7,10 à 7,60 (Mt, 15H aromatiques).

Le benzhydryloxycarbonyl-2 méthyl-3 oxo-8 phénylacétamido-7 oxa-5 aza-1 bicyclo[4.2.0] octène-2 peut être obtenu de la manière suivante:

On ajoute en 3 minutes une solution de 0,31 g de chlorure de phénylacétyle dans 1 $cm^3$ de chlorure de méthylène anhydre à un mélange de 0,73 g d'amino-7 benzhydryloxycarbonyl-2 méthyl-3 oxo-8 oxa-5 aza-1 bicyclo[4.2.0] octène-2 et de 0,2 g de triéthylamine dans 20 $cm^3$ de chlorure de méthylène anhydre maintenu à —20°C. On agite à cette température pendant 15 minutes, puis le mélange est transféré dans une ampoule à decanter où la phase chlorométhylénique est lavée successivement par 25 $cm^3$ d'une solution 0,1 N d'acide chlorhydrique, 25 $cm^3$ d'une solution demi-saturée de bicarbonate de sodium et 25 $cm^3$ d'eau distillée. Elle est séchée sur sulfate de magnésium anhydre. On filtre et lave le sulfate de magnésium par 5 $cm^3$ de chlorure de méthalène et les solutions organiques sont concentrées à sec sous pression réduite (100 mm de mercure; 13,5 kPa). On obtient 0,73 g de benzhydryloxycarbonyl-2 méthyle-3 oxo-8 phénylacétamido-7 oxa-5 aza-1 bicyclo[4.2.0] octène-2 sous la forme d'une meringue blanche.

Spectre de masse: pic moléculaire = 482.

Spectre infra-rouge (KBr), bandes caractéristiques ($cm^{-1}$: 3420, 3360, 1790, 1725, 1685, 1670, 1540, 1500, 1460, 1230, 750, 700.

Spectre de RMN du proton (350 MHz, $CDCl_3$, $\delta$ en ppm, J e Hz): 1,99 (s, 3H, $-CH_3$); 3,66 (s, 2H, $C_6H_5-CH_2-$); 4,17 (D, J = 17,5 1H, $-CH_2-O-$); 4,25 (D, J = 17,5, 1H, $-CH_2-O-$); 5,01 (D, J = 3,5, 1H, -H en 6); 5,68 (DD, J = 10 et 3,5, 1H, -H en 7); 6,16 (D, J = 10, 1H, -CONH-); 6,88 [s, 1H, $-COOC\underline{H}$ $(C_6H_5)_2]$; 7,13 à 7,60 (Mt, 15H aromatiques.

*Exemple 5*

Une solution de 0,09 g de benzhydryloxycarbonyl-2 (diméthylamino-2 vinyl)-3 oxo-8 phénoxyacétamido-7 oxa-5 aza-1 bicyclo[4.2.0] octène-2, forme E, dans 5 $cm^3$ d'acétate d'éthyle est agitée vigoureusement pendant 30 minutes à 20°C en présence de 3 $cm^3$ d'une solution d'acide chlorhydrique 1N. Le mélange est décanté et la phase organique est lavée par 10 $cm^3$ d'eau distillée, 10 $cm^3$ d'une solu-

tion à 5% de bicarbonate de sodium, 10 $cm^3$ d'eau distillée puis 10 $cm^3$ d'une solution demi-saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 0,07 g de benzhydryloxycarbonyl-2 (oxo-2 éthyl)-3 oxo-8 phénoxyacétamido-7 oxa-5 aza-1 bicyclo[4.2.0] octène-2 sous la forme d'une laque jaune.

Spectre de masse: pic moléculaire = 526.

Spectre infra-rouge (KBr), bandes caractéristiques ($cm^{-1}$): 3420, 2930, 2860, 2730, 1790, 1720, 1690, 1670, 1650, 1530, 1495, 1455, 1440, 1240, 1110, 1070, 760, 700.

Spectre de RMN du proton (350 MHz, $CDCl_3$, $\delta$ en ppm, J en Hz): 3,52 (D, J = 17,5, 1H, -C$\underline{H}_2$-CHO); 3,63 (d, J = 17,5, 1H, -C$\underline{H}_2$-CHO); 4,32 (d, J = 17,5, 1H, -$CH_2$-O-); 4,40 (D, J = 17,5, 1H, -$CH_2$-O-); 4,58 (s, 2H, $C_6H_5$ O-$CH_2$-); 5,15 (D, J = 3,5, 1H, -H en 6); 5,80 (DD, J = 10 et3,5, 1H, -H en 7); 6,88 [s, 1H, -COOC$\underline{H}$($C_6H_5$)$_2$]; 6,9 à 7,60 (Mt, 16H aromatiques et -CONH-); 9,60 (s, 1H, -C$\underline{H}$O).

Le benzhydryloxycarbonyl-2 (diméthylamino-2 vinyl)-3 oxo-8 phénoxyacétamido-7 oxa-5 aza-1 bicyclo[4.2.0] octène-2, forme E peut être obtenu de la manière suivante:

On chauffe à 80°C sous azote une solution de 0,86 g de benzhydryloxycarbonyl-2 méthyl-3 oxo-8 phénoxyacétamido-7 oxa-5 aza-1 bicyclo[4.2.0] octène-2 dans 13 $cm^3$ de diméthylformamide. On ajoute goutte à goutte en 2 minutes, dans la solution maintenue sous agitation à 80°C, 0,44 $cm^3$ de t.butoxy bis-diméthylaminométhane. L'addition terminée, le mélange est conservé sous agitation à 80°C pendant 20 minutes. La solution est diluée par 100 $cm^3$ d'acétate d'éthyle, la phase organique est lavée par trois fois 50 $cm^3$ d'eau distillée et 50 $cm^3$ d'une solution saturée de chlorure de sodium, séchée sur sulfate de magnésium, filtrée et concentrée à sec à 40°C sous pression réduite (20 mm de mercure; 2,7 kPa). On obtient 0,93 g d'un produit brut constitué principalement de benzhydryloxycarbonyl-2 (diméthylamino-2 vinyl)-3 oxo-8 phénoxyacétamido-7 oxa-5 aza-1 bicyclo[4.2.0] octène-2, forme E.

On purifie 0,90 g de ce produit par chromatographie sur une colonne de silice (0,04-0,06) (diamètre de la colonne: 3,8 cm; hauteur: 23 cm) en éluant sous 50 kPa avec un mélange de cyclohexane et d'acétate d'éthyle (40-60 en volumes) et en recueillant des fractions de 40 $cm^3$. Les fractions 9 à 13 sont rassemblées et concentrées à sec sous pression réduite (30 mm de mercure; 4 kPa) à 40°C. On obtient 0,12 g d'une meringue jaune orangé du produit pur.

Spectre infra-rouge (KBr), bandes caractéristiques ($cm^{-1}$): 3420, 1785, 1720, 1690, 1615, 1600, 1590, 1525, 1495, 1455, 1440, 1240, 1105, 1070, 765.

Spectre de RMN du proton (250 MHz, $CDCl_3$, $\delta$ en ppm, J en Hz): 2,86 [s, 6H, -N($CH_3$)$_2$]; 4,56 (AB, J = 14, 2H, $C_6H_5$-O-$CH_2$-); 4,60 (D, J = 16, 1H, -O-$CH_2$-); 4,79 (D, J = 16, 1H, -O-$CH_2$-); 5,08 (D, J = 4, 1H, H en -6); 5,70 (DD, J = 9 et 4, 1H, -H en 7); 6,36 (D, J = 14, 1H, -CH=CH-); 6,54 (D, J = 14, 1H, -CH=CH-); 6,88 [s, 1H, -COOC$\underline{H}$($C_6H_5$)$_2$]; 6,93 (D, J = 7,5, 2H, $C_6H_5$-O-$CH_2$-, H ortho); 7,03 (T, J = 7,5, 1H, $C_6H_5$ O$CH_2$-, H para); 7,10 à 7,70 (Mt, aromatiques et -CONH-).

Le benzhydryloxycarbonyl-2 méthyl-3 oxo-8 phénoxyacétamido-7 oxa-5 aza-1 bicyclo[4.2.0] octène-2 peut être préparé de la manière suivante:

On ajoute en 15 minutes une solution de 1,71 g de chlorure de phénoxyacétyle dans 5 $cm^3$ de chlorure de méthylène anhydre dans un mélange de 3,64 g d'amino-7 benzhydryloxycarbonyl-2 méthyl-3 oxo-8 oxa-5 aza-1 bicyclo[4.2.0] octène-2 et de 1,01 g de triéthylamine dans 50 $cm^3$ de chlorure de méthylène anhydre maintenu à —20°C. On agite à cette température pendant 15 minutes puis on enlève le bain réfrigérant et on laisse remonter la température du mélange vers 20°C. Le mélange est lavé successivement par 50 $cm^3$ d'une solution d'acide chlorhydrique 0,1 N, 50 $cm^3$ d'eau distillée, 50 $cm^3$ d'une solution demi-saturée de bicarbonate de sodium, 50 $cm^3$ d'eau distillée et 50 $cm^3$ d'une solution demi-saturée de chlorure de sodium. La solution chlorométhylénique est alors séchée sur sulfate de magnésium anhydre, filtrée et concentrée à sec sous pression réduite (100 mm de mercure; 13,5 kPa). On obtient un résidu meringué (4,73 g) constitué de benzhydryloxycarbonyl-2 méthyl-3 oxo-8 phénylacétamido-7 oxa-5 aza-1 bicyclo[4.2.0] octène-2.

Spectre de masse: pic moléculaire = 498.

Spectre infra-rouge (KBr), bandes caractéristiques ($cm^{-1}$: 3420, 3350, 1790, 1720, 1690, 1640, 1600, 1590, 1530, 1495, 1490, 1455, 1440, 1230, 1110, 1065, 755,, 700.

Spectre de RMN du proton (350 MHz, $CDCl_3$, $\delta$ en ppm, J en Hz): 2,04 (s, 3H, -$CH_3$); 4,34 (s, 2H, -$CH_2$O-); 4,58 (s, 2H, $C_6H_5$O-C$\underline{H}_2$-); 5,11 (D, J = 3,5, 1H, -H en 6); 5,75 (DD, J = 10,5 et 3,5, 1H, -H en 7); 6,92 [s, 1H, -COOC$\underline{H}$($C_6H_5$)$_2$]; 6,94 [D, J = 7,5, 2H, $C_6H_5$O- (H ortho)]; 7,04 [t, J = 7,5, 1H, $C_6H_5$O- (H para)]; 7,20 à 7,60 (Mt, 13H aromatiques et -CO-N$\underline{H}$-).

*Exemple 6*

Une solution de 0,010 g de benzhydryloxycarbonyl-2 (diméthylamino-2 vinyl)-3 méthoxy-7$\alpha$ oxo-8 phénoxyacétamido-7 oxa-5 aza-1 bicyclo[4.2.0] octène-2, forme E, dans 5 $cm^3$ d'acétate d'éthyle est agitée vigoureusement pendant 30 minutes à 20°C en présence de 3 $cm^3$ d'une solution d'acide chlorhydrique 1N. Le mélange est décanté et la phase organique est lavée par 10 $cm^3$ d'eau distillée, 10 $cm^3$ d'une solution saturée de bicarbonate de sodium, 10 $cm^3$ d'eau distillée et 10 $cm^3$ d'une solution saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 0,009 g de benzhydryloxycarbonyl-2 méthoxy-7$\alpha$ (oxo-2 éthyl)-3 oxo-8 phénoxyacétamido-7 oxa-5 aza-1 bicyclo[4.2.0] octène-2, sous la forme d'une meringue marron clair.

Spectre de masse: pic moléculaire = 556.

Spectre de RMN du proton (350 MHz, $CDCl_3$, $\delta$ en ppm, J en Hz): 3,50 (s, -$OCH_3$); 4,25 (s, -O-$CH_2$-); 4,50 (s, $C_6H_5$O-C$\underline{H}_2$-); 5,08 (s, $H_6$); 6,79 [s, -COOCH($C_6H_5$)$_2$]; 9,48 (s, -CHO).

Le benzhydryloxycarbonyl-2 (diméthylamino-2 vinyl)-3 méthoxy-7α oxo-8 phénoxyacétamido-7 oxa-5 aza-1 bicyclo[4.2.0] octène-2, forme E peut être obtenu de la manière suivante:

On chauffe à 80°C sous azote, une solution de 1,5 g de benzhydryloxycarbonyl-2 méthoxy-7α méthyl-3 oxo-8 phénoxyacétamido-7 oxa-5 aza-1 bicyclo[4.2.0] octène-2 dans 0,7 $cm^3$ de diméthylformamide. Dans la solution maintenue sous agitation à 80°C, on ajoute goutte à goutte en 5 minutes, 0,7 $cm^3$ de t.butoxycarbonyl- bis- diméthylaminométhane. L'addition terminée, le mélange est conservé sous agitation à 80°C pendant 30 minutes. La solution est diluée par 100 $cm^3$ d'acétate d'éthyle, la phase organique est lavée par six fois 100 $cm^3$ d'eau distillée et 100 $cm^3$ d'une solution saturée de chlorure de sodium, séchée sur sulfate de magnésium, filtrée et concentrée à sec sous pression réduite (20 mm de mercure; 2,7 kPa). On obtient 1,19 g d'un produit brut constitué principalement de benzhydryloxycarbonyl-2 (diméthylamino-2 vinyl)-3 méthoxy-7α oxo-8 phénoxyacétamido-7 oxa-5 aza-1 bicyclo[4.2.0] octène-2, forme E.

Spectre de masse: pic moléculaire = 583.

Spectre infra-rouge (KBr), bandes caractéristiques ($cm^{-1}$: 1775, 1700, 1615, 1495, 1460, 1440, 760, 700.

Spectre de RMN du proton (350 MHz, $CDCl_3$, δ en ppm, J en Hz): 2,85 [s, 6H, $-N(CH_3)_2$]; 3,58 (s, 3H, $CH_3O$-); 4,57 (s, 2H, $-O-CH_2-$); 4,63 (s, 2H, $C_6H_5$ $OCH_2$-); 5,14 (s, 1H, -H en 6); 6,30 (D, J = 14, 1H, -CH=CH-); 6,53 (D, J = 14, 1H, -CH=CH-); 6,88 [s, 1H, $-COOC\underline{H}(C_6H_5)_2$]; 6,9 à 7,6 (Mt, 15H aromatiques).

Le benzhydryloxycarbonyl-2 méthoxy-7α méthyl-3 oxo-8 phénoxyacétamido-7 oxa-5 aza-1 bicyclo[4.2.0] octène-2 peut être obtenu de la manière suivante:

On ajoute 13 $cm^3$ d'une solution 1,6 N de n.butyllithium dans l'hexane à 50 $cm^3$ de tétrahydrofuranne sec refroidi à —40°C et maintenu sous azote. On ajoute lentement 3 $cm^3$ de méthanol anhydre, puis on refroidit le mélange à —74°C et on ajoute une solution de 3 g de benzhydryloxycarbonyl-2 méthyl-3 oxo-8 phénoxyacétamido-7 oxa-5 aza-1 bicyclo[4.2.0] octène-2 dans 14 $cm^3$ de tétrahydrofuranne. La solution obtenue est maintenue à —74°C pendant 3 minutes et on ajoute 0,84 $cm^3$ d'hypochlorite de t.butyle. Le mélange est maintenu sous agitation à —74°C pendant 1 heure et 20 minutes, puis on verse en filet 2 $cm^3$ d'un mélange d'acide acétique glacial et de triéthylphosphite (50-50 en volumes). On laisse la température du mélange réactionnel remonter vers 20°C et on dilue par 100 $cm^3$ d'acétate d'éthyle. La phase organique est lavée successivement par 100 $cm^3$ d'une solution de bicarbonate de sodium à 5%, 100 $cm^3$ d'eau distillée et 100 $cm^3$ d'une solution saturée de chlorure de sodium, séchée sur sulfate de magnésium anhydre, filtrée et le solvant est évaporé sous 20 mm de mercure (2,7 kPa). Le résidu est purifié par chromatographie sur gel de silice (0,04-0,06) (diamètre de la colonne: 4,2 cm; hauteur de silice: 24 cm) en éluant par un mélange de cyclohexane et d'acétate d'éthyle (55-45) en volumes) sous une pression de 40 kPa et en recueillant des fractions de 50 $cm^3$. Les fractions 10 à 17 sont concentrées à sec sous pression réduite (30 mm de mercure; 4 kPa) à 30°C. On obtient 1,55 g de benzhydryloxycarbonyl-2 méthoxy-7α méthyl-3 oxo-8 phénoxyacétamido-7 oxa-5 aza-1 bicyclo-[4.2.0] octène-2 sous la forme d'une meringue blanche.

Spectre de masse: pic moléculaire = 528.

Spectre infra-rouge ($CHBr_3$), bandes caractéristiques ($cm^{-1}$: 3410, 1780, 1710, 1645, 1600, 1590, 1500, 1460, 1440, 1230, 1080, 760.

Spectre de RMN du proton (350 MHz, $CDCl_3$, δ en ppm, J en Hz): 2,02 (s, 3H, $-CH_3$); 3,58 (s, 3H, $-OCH_3$); 4,27 (D, J = 17,5, 1H, $-OCH_2-$); 4,34 (D, J = 17,5, 1H, $-O-CH_2-$); 4,58 (s, 2H, $C_6H_5-O-CH_2-$); 5,14 (s, 1H, -H en 6); 6,92 [s, 1H, $-COOC\underline{H}(C_6H_5)_2$]; 6,96 (D, J = 7, 2H, $C_6H_5-O-$, H ortho); 7,05 (T, J = 7, 1H, H en para de $C_6H_5O$-); 7,15 à 7,60 (Mt, H aromatiques + -CONH-).

*Exemple 7*

Une solution de 0,016 g de (diméthylamino-2 vinyl)-3 p.nitrobenzyloxycarbonyl-2 oxo-8 tritylamino-7 oxa-5 aza-1 bicyclo[4.2.0] octène-2, forme E (obtenu comme décrit ci-après) dans 5 $cm^3$ d'acétate d'éthyle est agitée vigoureusement pendant 30 minutes à 20°C en présence de 5 $cm^3$ d'une solution d'acide chlorhydrique 1N. Le mélange est décanté et la phase organique est lavée par 2 fois 5 $cm^3$ d'une solution demi-saturée de bicarbonate de sodium puis par 2 fois 5 $cm^3$ d'une solution demi-saturée de chlorure de sodium. La phase acétate d'éthyle est séchée sur sulfate de sodium anhydre, filtrée et concentrée à sec à 30°C sous pression réduite (20 mm de mercure; 2,7 kPa). On obtient 0,012 g de p.nitrobenzyloxycarbonyl-2 (oxo-2 éthyl)-3 oxo-8 tritylamino-7 oxa-5 aza-1 bicyclo[4.2.0] octène-2, sous la forme d'une meringue marron clair.

Spectre de RMN du proton (250 MHz, $CDCl_3$, δ en ppm, J en Hz): 3,44 (D, J = 16,25, 1H, $-C\underline{H}_2-CHO$); 3,57 (D, J = 16,25, 1H, $-C\underline{H}_2-CHO$); 3,84 (D, J = 3,75, 1H, -H en 6); 3,96 (D, J = 18,75, 1H, $-CH_2-O-$); 4,17 (D, J = 18,75, 1H, $-CH_2O-$); 4,34 (DD, J = 9 et 3,75, 1H, -H en 7); 5,21 (D, J = 12,0, 1H, $-C\underline{H}_2C_6H_4NO_2$); 5,37 (D, J = 12,0, 1H, $-C\underline{H}_2$ $C_6H_4NO_2$); 7,10 à 7,75 [Mt, 18H aromatiques et $(C_6H_5)_3$ C-NH-]; 8,23 (D, J = 8, 2H aromatiques en ortho du nitro); 9,56 (s, 1H, $-C\underline{H}O$).

Le diméthylamino-2 vinyl-3 p.nitrobenzyloxycarbonyl-2 oxo-8 tritylamino-7 oxa-5 aza-1 bicyclo-[4.2.0] octène-2, forme E peut être obtenu de la manière suivante:

On chauffe à 70°C sous azote, une solution de 0,58 g de méthyl-3 p.nitrobenzyloxycarbonyl-2 oxo-8 tritylamino-7 oxa-5 aza-1 bicyclo[4.2.0] octène-2 dans 10 $cm^3$ de dioxanne sec. On ajoute goutte à goutte en 10 minutes une solution de 0,5 $cm^3$ de t.butoxy bis-diméthylaminométhane dans 2 $cm^3$ de dioxanne sec. L'addition terminée, le mélange est conservé sous agitation à 70°C pendant 20 minutes. La solution est diluée par 100 $cm^3$ d'acétate d'éthyle, la phase organique est lavée par six fois 100 $cm^3$ d'une solution demi-saturée de chlorure de sodium, séchée sur sulfate de sodium, filtrée et concentrée à sec à 40°C sous pression réduite (20 mm de mercure; 2,7 kPa). On obtient 0,2 g d'un produit brut que l'on purifie par chromatographie sur une colonne de silice (0,04-0,06) (diamètre de la colonne: 2,1 cm; hauteur: 20 cm) en éluant sous 50 kPa avec un mélange de cyclohexane et d'acétate d'éthyle (60-40 en volumes) et en recueillant des fractions de 15 $cm^3$. La fraction 9 est concentrée sec sous pression réduite (30 mm de mercure; 4 kPa) à 40°C. On obtient 0,02 g d'une poudre jaune se présentant comme un mélange de (diméthylamino-2 vinyl)- p.nitrobenzyloxycarbonyl-2 oxo-8 tritylamino-7 oxa-5 aza-1 bicyclo[4.2.0] octène-2, forme E et du dérivé (oxo-2 éthyl)-3 correspondant.

Spectre de RMN du proton (250 MHz, $CDCl_3$, δ en ppm, J en Hz: 2,83 [s, 6H, $-N(CH_3)_2$]; 3,75 (D, J = 3, 1H, -H en 6); 4,21 (D, J = 15,5, 1H, $-O-CH_2-$); 4,43 (DD, J = 9 et 3, 1H, -H en 7); 4,56 (D, J = 15,5, 1H, $-O-CH_2-$); 5,18 (D, J = 12,0, 1H, $-CH_2-C_6H_4NO_2$); 5,41 (D, J = 12,0, 1H, $-CH_2-C_6H_4NO_2$); 6,21 (D, J = 13,75, 1H, -CH=CH-); 6,48 (D, J = 13,75, 1H, -CH=CH-); 7,10 à 7,75 [Mt, 18H aromatiques et $(C_6H_5)_3$ C-NH-]; 8,20 (D, J = 8, 2H aromatiques en ortho du nitro).

*Exemple de reference 1*

On dissout 2,51 g de benzhydryloxycarbonyl-2 (oxo-2 éthyl)-3 oxo-8 tritylamino-7 oxa-5 aza-1 bicyclo[4.2.0] octène-2 dans 20 $cm^3$ de pyridine. A la solution obtenue, on ajute 1,13 g de chlorure de tosyle et on maintient le mélange sous agitation à 20°C pendant 1 heure 25 minutes. La solution est versée dans 150 $cm^3$ d'eau glacée, une gomme se dépose sur les parois du récipient, la phase aqueuse est décantée et on dissout la substance gommeuse dans 45 $cm^3$ d'acétate d'éthyle. La solution organique est lavée par 2 fois 50 $cm^3$ d'une solution 0,1 N d'acide chlorhydrique, 50 $cm^3$ d'une solution à 5% de bicarbonate de sodium et 30 $cm^3$ d'une solution demi-saturée de chlorure de sodium, séchée sur sulfate de sodium, filtrée et concentrée à sec à 30°C sous pressionréduite (20 mm de mercure; 2,7 kPa). On recueille 2,4 g d'un produit brut marron constitué essentiellement de benzhydryloxycarbonyl-2 oxo-8 (tosyloxy-2 vinyl)-3 tritylamino-7 oxa-5 aza-1 bicyclo-[4.2.0] octène-2, mélange des formes E et Z.

Spectre infra-rouge (KBr), bandes caractéristiques ($cm^{-1}$: 1790, 1725, 1595, 1490, 1450, 1380, 1190, 1180, 745, 700.

A une solution de 2,4 g de benzhydryloxycarbonyl-2 oxo-8 (tosyloxy-2 vinyl)-3 tritylamino-7 oxa-5 aza-1 bicyclo[4.2.0] octène-2 mélange des formes E et Z, dans 15 $cm^3$ de diméthylformamide, on ajoute 0,28 g de sel de sodium de mercapto-5 méthyl-2 thiadiazole-1,3,4. Le mélange est agité à 20°C pendant 2 heures, puis est dilué par 50 $cm^3$ d'acétate d'éthyle. La solution est lavée par 5 fois 50 $cm^3$ d'eau distillée et 50 $cm^3$ d'une solution demi-saturée de chlorure de sodium, puis séchée sur sulfate de sodium. On filtre et concentre à sec à 20°C sous pression réduite (20 mm de mercure; 2,7 kPa). Le résidu (2,1 g) est chromatographié sur une colonne de gel de silice Merck (0,04-0,06) (diamètre de la colonne: 4,1 cm; hauteur: 20 cm). On élue par 1 litre d'un mélange de cyclohexane-acétate d'éthyle 70 à 30 (en volumes) sous une pression de 50 kPa en recueillant des fractions de 60 $cm^3$. Les fractions 9 et 10 sont concentrée à sec sous pression réduite (30 mm de mercure; 4 kPa) à 30°C. On obtient 0,13 g de benzhydryloxycarbonyl-2 [(méthyl-2 thiadiazol--1,3,4 yl-5) thio-2 vinyl]-3 oxo-8 tritylamino-7 oxa-5 aza-1 bicyclo[4.2.0] octène-2, forme E sous la forme d'une meringue jaune pâle.

Spectre infra-rouge (KBr), bandes caractéristiques ($cm^{-1}$: 1790, 1720, 1490, 1450, 1210, 745, 700.

Spectre de RMN du proton (350 MHz, $CDCl_3$, δ en ppm, J en Hz): 2,74 (s, 3H, $-CH_3$); 3,76 (d, J = 3,5, 1H, H en 6); 4,16 et 4,62 (2d, J = 18, 2H, $-CH_2-O-$); 4,37 (d, J = 3,5, 1H, H en 7); 6,84 [s, 1H, -COO-$C\underline{H}(C_6H_5)_2$];6,96 (d, J = 17, 1H, $-C\underline{H}=CH-S-$).

On dissout 0,112 g de benzhydryloxycarbonyl-2 [(méthyl-2 thiadiazol-1,3,4 yl-5) thio-2 vinyl]-3 oxo-8 tritylamino-7 oxa-5 aza-1 bicyclo[4.2.0] octène-2, forme E dans 1,4 $cm^3$ d'acétone et on ajoute à la solution 0,029 g d'acide p.toluènesulfonique monohydraté. La solution est portée au reflux pendant 45 minutes pendant lesquelles des cristaux se développent sur les parois du récipient. La suspension est filtrée et le filtrat est versé dans 10 $cm^3$ d'une solution à 1% de bicarbonate de sodium. Le mélange est extrait par 2 fois 5 $cm^3$ d'acétate d'éthyle, la phase organique est séchée sur sulfate de sodium, filtrée et concentrée à sec à 30°C sous pression réduite (20 mm de mercure; 2,7 kPa). On recueille ainsi 0,1 g d'un solide brut marron constitué essentiellement d'amino-7 benzhydryloxycarbonyl-2 [(méthyl-2 thiadiazol-1,2,3 yl-5) thio-2 vinyl]-3 oxo-8 oxa-5 aza-1 bicyclo[4.2.0] octène-2, forme E.

Rf = 0,20 [chromatoplaque de silicagel, éluant: cyclohexane-acétate d'éthyle 80-20 (en volumes)].

A une solution de 0,1 g d'amino-7 benzhydryloxycarbonyl-2 [(méthyl-2 thiadiazol-1,3,4 yl-5) thio-2 vinyl]-3 oxo-8 oxa-5 aza-1 bicyclo[4.2.0] octène-2, forme E dans 20 $cm^3$ de dichlorométhane, on ajoute 0,089 g d'acide méthoxyimino-2 (tritylamino-2 thiazolyl-4)-2 acétique, isomère syn, 0,041 g de N,N'-dicyclohexylcarbodiimide et 0,001 g de N,N-diméthylamino-4 pyridine. Le mélange est agité à 20°C pendant 1 heure 30. On ajoute alors 0,1 $cm^3$ d'acide acétique; on élimine un léger insoluble par filtration, concentre à sec le filtrat à 30°C sous pression réduite (100 mm de mercure; 13 kPa) et dissout le résidu dans 5 $cm^3$ d'acétate d'éthyle. La solution est lavée par 2 folis 2,5 $cm^3$ d'acide chlorhydrique 0,1 N, puis par 5 $cm^3$ d'une solution à 1% de bicarbonate de sodium, et par 5 $cm^3$ d'eau distillée. On sèche sur sulfate de sodium, filtre et concentre à sec à 30°C sous 20 mm de mercure (2,7 kPa). Le résidu (0,12 g) est fixé sur 0,25 g de gel de silice Merck (0,05-0,2) et la poudre obtenue est déposée sur une colonne de 10 g de gel de silice (diamètre de la colonne: 1 cm). On élue successivement par 70 $cm^3$ d'un mélange cyclohexane-acétate d'éthyle 50-50 (en volumes), 30 $cm^3$ d'un mélange 40-60 et 30 $cm^3$ d'un mélange 20-80 en recueillant des fractions de 2,5 $cm^3$. On concentre à sec à 20°C sous pression réduit (20 mm de mercure; 2,7 kPa) les fractions 22 à 40 et recueille 0,023 g de benzhydryloxycarbonyl-2 [méthoxyimino-2 (tritylamino-2 thiazolyl-4)-2 acétamido]-7 [(méthyl-2 thiadiazol-1,3,4 yl-5) thio-2 venyl]-3 oxo-8 oxa-5 aza-1 bicyclo[4.2.0] octène-2 isomère syn, forme E sous la forme d'une meringue orangée.

Spectre infra-rouge (KBr), bandes caractéristiques (cm$^{-1}$: 1795, 1720, 1685, 1520, 1495, 1450, 1210, 1045, 750, 700.

Spectre de RMN du proton (350 MHz, $CDCl_3$, δ en ppm, J en Hz): 2,74 (s, 3H, $-CH_3$); 4,07 (s, 3H, $=N-O-CH_3$); 4,59 et 4,86 (2d, J = 18, 2H, $-CH_2-O-$); 5,13 (d, J = 3,5, 1H, -H en 6); 5,81 (dd, J = 3,5 et 9, 1H, -H en 7); 6,78 (s, 1H, -H du thiazole); 6,79 (d, J = 9, 1H, -CO-NH-); 6,88 [s, 1H, $-COO-C\underline{H}(C_6H_5)_2$]; 7,03 [mf, 1H, $-N\underline{H}-C(C_6H_5)_3$]; 7,14 et 7,69 (2d, J = 17, 2H, -CH=CH-S-).

A une solution de 0,023 g de benzhydryloxycarbonyl-2 [méthoxyimino-2 (tritylamino-2 thiazolyl-4)-2 acétamido]-7 [(méthyl-2 thiadiazol-1,3,4 yl-5) thio-2 vinyl]-3 oxo-8 oxa-5 aza-1 bicyclo[4.2.0] octène-2, isomère syn, forme E, dans 1 cm$^3$ d'acide formique, on ajoute 0,5 cm$^3$ d'eau distillée et chauffe le mélange à 50°C sous agitation pendant 20 minutes. Après refroidissement, on filtre l'insoluble et on concentre à sec à 30°C sous pression réduite (20 mm de mercure; 2,7 kPa). On reprend le résidu par 2 fois 15 cm$^3$ d'éthanol en concentrant à sec à chaque fois à 20°C sous 20 mm de mercure (2,7 kPa) et triture le résidu dans 20 cm$^3$ d'éther éthylique. On obtient après filtration 0,010 g d'[(amino-2 thiazolyl-4)-2 méthoxyimino-2 acétamido]-7 carboxy-2 [(méthyl-2 thiadiazol-1,3,4 yl-5) thio-2 vinyl]-3 oxo-8 oxa-5 aza-1 bicyclo[4.2.0] octène-2, isomère syn, forme E sous la forme d'une poudre jaune.

Spectre infra-rouge (KBr), bandes caractéristiques (cm$^{-1}$: 1785, 1670, 1620, 1530, 1040.

Spectre de RMN du proton (350 MHz, DMSO $d_6$, δ en ppm, J en Hz): 2,70 (s, $-CH_3$); 3,83 (s, $=N-OCH_3$); 4,50 et 4,78 (2d, J = 18, $-CH_2-O-$); 5,14 (d, J = 3,5, -H en 6); 5,45 (mf, -H en 7); 6,75 (s, -H du thiazole); 7,10 à 7,70 (mt, $-NH_2$ et -CH=CH-S-); 9,34 (d, J = 9, -CO-NH-).

*Exemple de reference 2*

Le produit de l'exemple 2 peut être utilisé de la manière suivante:

On ajoute en 10 minutes 0,27 cm$^3$ de triéthylamine à une solution refoidie à —5°C de 1,00 g de benzhydryloxycarbonyl-2 [méthoxyimino-2 (tritylamino-2 thiazolyl-4)-2 acétamido]-7 oxo-8 (oxo-2 éthyl)-3 oxa-5 aza-1 bicyclo[4.2.0] octène-2, isomère syn, et de 0,35 g de chlorure de tosyle dans 20 cm$^3$ de dichlorométhane. On laisse sous agitation à —5°C pendant 10 minutes puis on laisse remonter à 20°C. Le mélange est alors agité pendant 30 minutes. Le solvant est évaporé sous pression réduite (350 mm de mercure; 47 kPa). On obtient ainsi une huile marron qui se solidifie par agitation avec 100 cm$^3$ d'éther. Le solide est séparé sur filtre, et séché. On obtient 0,38 g de benzhydryloxycarbonyl-2 [méthoxyimino-2 (tritylamino-2 thiazolyl-4)-2 acétamido]-7 oxo-8 (tosyloxy-2 vinyl)-3 oxa-5 aza-1 bicyclo[4.2.0] octène-2, isomère syn, forme E, sous la forme d'une poudre de couleur crème. Le filtrat est concentré jusqu'à un volume résiduel de 5 cm$^3$, et on ajoute 100 cm$^3$ d'éther isopropylique. Un solide jaune pâle se sépare. On l'isole sur filtre et obtient 0,11 g d'une deuxième fraction de benzhydroxyloxycarbonyl-2 [méthoxyimino-2 (tritylamino-2 thiazolyl-4)-2 acétamido]-7 oxo-8 (tosyloxy-2 vinyl)-3 oxa-5 aza-1 bicyclo[4.2.0] octène-2, isomère syn, forme E.

Spectre infra-rouge (KBr), bandes caractéristiques (cm$^{-1}$: 3400, 1800, 1720, 1685, 1600, 1520, 1495, 1450, 1380, 1195, 1180, 1070, 815, 755, 700.

Spectre de RMN du proton (250 MHz, $CDCl_3$, δ en ppm, J en Hz): 2,44 (s, 3H, $CH_3$ tosyl); 4,08 (s, 3H, $=NOCH_3$); 4,44 (D, J = 16,25, 1H, $-O-CH_2-$); 4,64 (D, J = 16,25, 1H, $-O-CH_2-$); 5,12 (D, J = 3,75, 1H, -H en 6); 5,79 (DD, J = 8 et 3,75, -1H, -H en 7); 6,72 (D, J = 8, 1H, -CONH-); 6,77 )s, 1H, -H du thiazole); 6,82 (D, J = 12,5, 1H, -CH=CH-); 6,83 [s, 1H, $-COOCH(C_6H_5)_2$]; 7,03 [Mf, 1H, $(C_6H_5)_3 CN\underline{H}-$); 7,17 (D, J = 12,5, 1H, -CH=CH-); 7,20 à 7,55 (Mf, 27H aromatiques); 7,75 (D, J = 8, 2H ortho du tosyle).

Une solution de 0,53 g de benzhydryloxycarbonyl-2 [méthoxyimino-2 (tritylamino-2 thiazolyl-4)-2 acétamido-7 oxo-8 (tosyloxy-2 vinyl)-3 oxa-5 aza-1 bicyclo[4.2.0] octène-2, isomère syn, forme E, et de 0,139 g de [(diméthoxy-2,2 éthyl)-4 dioxo-5,6 tétrahydro-1,4,5,6 triazine-1,2,4 yl-3] thiolate de sodium dans 12 cm$^3$ de diméthylformamide est portée à 40°C sous agitation pendant 6 heures. Le mélange est transféré dans une ampoule à décanter contenant 30 cm$^3$ d'acétate d'éthyle et 50 cm$^3$ d'eau distillée. Après décantation la phase organique est lavée par 3 fois 50 cm$^3$ d'eau distillée puis séchée sur sulfate de magnésium anhydre. Le solvant est évaporé sous pression réduite (20 mm de mercure; 2,7 kPa) et le résidu (0,52 g) est purifié par chromatographie sur une colonne de gel de silice Merck (0,04-0,06) (diamètre de la colonne: 2,2 cm; hauteur: 25,5 cm). On élue par un mélange de cyclohexane-acétate d'éthyle 15-85 (en volumes) sous une pression de 50 kPa en recueillant des fractions de 20 cm$^3$. Les fractions 10 à 28 sont concentrées à sec sous pression réduite (30 mm de mercure; 4 kPa) à 30°C. On obtient 0,20 g de benzhydryloxycarbonyl-2 {[(diméthoxy-2,2 éthyl)-4 dioxo-5,6 tétrahydro-1,4,5,6 triazine-1,2,4 yl-3] thio-2 vinyl}-3 [méthoxyimino-2 (tritylamino-2 thiazolyl-4) acétamido]-7 oxo-8 oxa-5 aza-1 bicyclo[4.2.0] octène-2, isomère syn, forme E, sous la forme d'une meringue marron clair.

Spectre de RMN du proton (250 MHz, $CDCl_3$, δ en ppm, J en Hz): 3,42 [s, 6H, $(-OCH_3)_2$]; 4,03 (AB, limite, J = 4,5 et 10, 2H, $>NCH_2-$); 4,06 (s, 3H, $=N-OCH_3$); 4,58 (D, J = 16,25, 1H, $-OCH_2-$); 4,65 [T, J = 5, 1H, $-C\underline{H}(OCH_3)_2$]; 4,84 (D, J = 16,25, 1H, $-O-CH_2-$); 5,15 (D, J = 3,15, 1H, -H en 6); 5,81 (DD, J = 10 et 3,15, 1H, -H en 7); 6,73 (D, J = 16,25, 1H, -CH=CH-); 6,79 (s, 1H, -H du thiazole); 6,87 [s, 1H, $-COO\ C\underline{H}(C_6H_5)_2$]; 7,05 [Mf, 1H, $(C_6H_5)_3CN\underline{H}-$]; 7,18 (D, J = 10, 1H, -CONH-); 7,20 à 7,55 (Mf, 25H aromatiques); 7,68 (D, J = 16,25, 1H, -CH=CH-); 10,09 (s large, 1H, -NH- triazine).

On ajoute 0,74 g d'acide p.toluènesulfonique monohydraté à une solution de 0,20 g de benzhydryloxycarbonyl-2 {[(diméthoxy-2,2 éthyl)-4 dioxo-5,6 tétrahydro-1,4,5,6 triazine-1,2,4 yl-3] thio-2 vinyl}-3 [méthoxyimino-2 (tritylamino-2 thiazolyl-4)-2 acétamido]-7 oxo-8 oxa-5 aza-1 bicyclo[4.2.0] octène-2, isomère syn, forme E dans 5 cm$^3$ d'acétonitrile à 50°C. Le mélange est maintenu à cette température pendant 30 minutes. Après refroidissement à 20°C, on filtre le précipité qui s'est développé au cours de la réaction.

Il est lavé par 1 $cm^3$ d'acétonitrile, puis il est agité vigoureusement dans 5 $cm^3$ d'eau distillée pendant 30 minutes. La suspension est filtrée, et la poudre marron obtenue est séchée sous pression réduite (10 mm de mercure; 1,33 kPa) donnant 20 mg de tosylate d'[amino-2 thiazolyl-4)-2 méthoxyimino-2 acétamido]-7 carboxy-2{[dioxo-5,6 (oxo-2 éthyl)-4 tétrahydro-1,4,5,6 triazine-1,2,4 yl-3] thio-2 vinyl}-3 oxo-8 oxa-5 aza-1 bicyclo[4.2.0] octène-2, isomère syn, forme E.

Spectre de RMN du proton (250 MHz, $CF_3CO_2D$, $\delta$ en ppm, J en Hz): 2,47 (s, 3H, $-CH_3$); 4,30 (s, 3H, $=NOCH_3$); 5,17 (Mf, $\langle NCH_2-$); 5,45 (Mf, 1H, -H en 6); 5,91 (Mf, 1H, -H en 7); 7,39 (D, J = 8, 2H, H tosyle); 7,53 (s, 1H, -H du thiazole); 7,82 (D, J = 8, 2H, H tosyl); 9,76 (s, 1H, -CHO).

**Revendications pour les Etats Contractants:**
BE, CH, DE, FR, GB, IT, LI, LU, NL, SE

1. Une nouvelle oxacéphalosporine caractérisée en ce qu'elle répond à la formule générale:

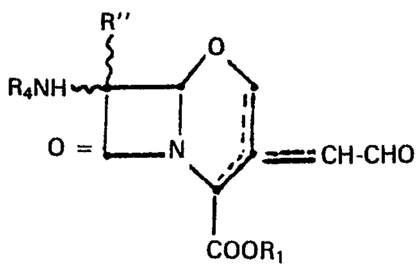

qui se présente sous forme bicyclooctène-2 ou -3 oxoéthylidène-3 bicyclooctane, dans laquelle le symbole $R_1$ représente un radical protecteur, et

a) le symbole $R_4$ représente un radical de formule générale:

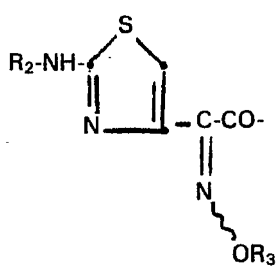

[dans laquelle $R_2$ est un radical protecteur d'amino, $R_3$ est un atome d'hydrogène, un raidcal protecteur, un radical alcoyle, vinyle, ou carboxyalcoyle représenté par la formule générale:

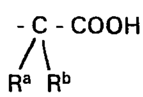

dans laquelle les radicaux $R^a$ et $R^b$, qui sont identiques ou différentes, représentent des atomes d'hydrogène ou des radicaux alcoyle, ou forment ensemble un radical alcoylène contenant 2 ou 3 atomes de carbone et dont la fonction acide peut être protégée], ou $R_4$ représente un radical $\alpha$-carboxyarylacétale libre ou protégé dans lequel aryle représente phényle, p.hydroxy phényle libre ou protégé, ou thiényle-2 ou -3, et

le symbole R'' représente un atome d'hydrogène ou un radical méthoxy en position 7$\alpha$, ou bien

b) le symbole $R_4$ est un radical facilement éliminable et le symbole R'' est un atome d'hydrogène ou un radical méthoxy en 7$\alpha$, ou un atome d'hydrogène en 7$\beta$, étant entendu que les radicaux et portions alcoyles cités ci-dessus sont (sauf mention spéciale) droits ou ramifiés et contiennent 1 à 4 atomes de carbone, le cas échéant sous ses formes syn ou anti.

2. Un nouveau produit selon la revendication 1, caractérisé en ce que $R_4$ représente un radical de formule générale:

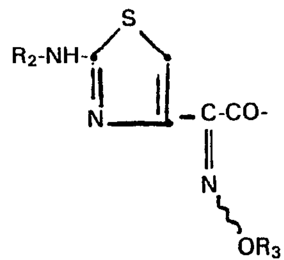

dans laquelle $R_3$ est défini selon la revendication 1, et $R_2$ est un groupement t.butoxycarbonyle, trichloro-2,2,2 éthoxycarbonyle, trichloracétyle, chloracétyle, trityle, dibenzyle, benzyle, benzyloxycarbonyle, p.nitrobenzyloxycarbonyle, p.méthoxybenzyloxycarbonyle, formyle ou trifluoroacétyle.

3. Un nouveau produit selon la revendication 1, caractérisé en ce que $R_4$ représente un radical de formule générale:

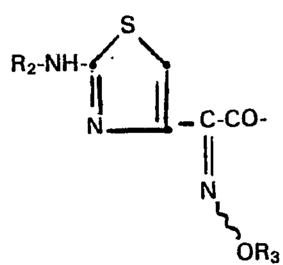

dans laquelle $R_2$ est défini comme dans la revendication 1 et $R_3$ est un groupement trityle, tétrahydropyrannyle ou méthoxy-2 propyle-2 protecteur de la fonction oxime.

4. Un nouveau produit selon la revendication 1, caractérisé en ce que $R_4$ est un radical de formule générale:

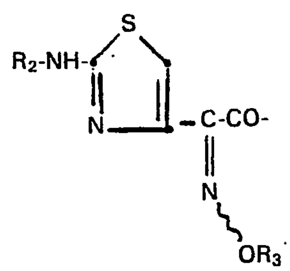

dans laquelle $R_2$ est défini comme dans la revendication 1 et $R_3$ est un groupement carboxyalcoyle [représenté par la formule générale:

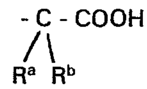

dans laquelle $R^a$ et $R^b$ sont définis comme dans la revendication 1] préalablement protégé par un radical choisi parmi méthoxyméthyle, t.butyle, benzhydryle, benzyle, nitrobenzyle et p.méthoxybenzyle.

5. Un nouveau produit selon la revendication 1, caractérisé en ce que $R_1$ est un radical facilement éliminable choisi parmi les radicaux méthoxyméthyle, t.butyle, benzhydryle, benzyle, nitrobenzyle et p.méthoxybenzyle.

6. Un procédé de préparation d'un produit selon la revendication 1, caractérisé en ce que l'on hydrolyse une énamine (ou un mélange d'énamines isomères) de formule générale:

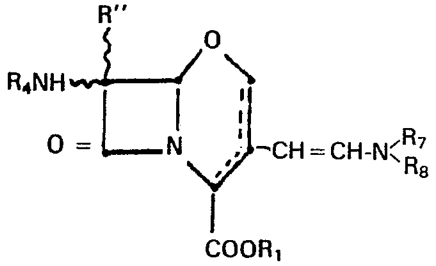

[dans laquelle $R_1$, $R_4$ et R'' sont définis comme dans la revendication 1 et $R_7$ et $R_8$, qui sont identiques ou différents, représentent des radicaux alcoyle (éventuellement substitués par un radical alcoyloxy ou dialcoylamino) ou phényle, ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle étant entendu que les radicaux et portions alcoyles cités ci-dessus sont droits ou ramifiés et contiennent 1 à 4 atomes de carbone], qui se présente sous forme bicyclooctène-2 ou -3, et dont le substituant sur l'atome de carbone en position -3 présente la stéréoisomérie E ou Z, puis on élimine éventuellement les groupements protecteurs et on sépare éventuellement les isomères du produit obtenu.

**Reventication pour l'Etat Contractant:** AT

Procédé de préparation d'une nouvelle oxacéphalosporine de formule générale

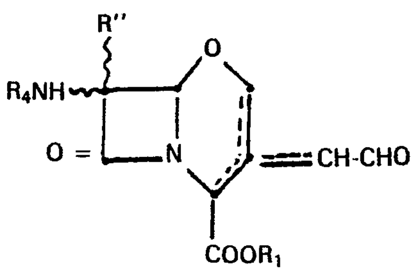

qui se présente sous forme bicyclooctène-2 ou -3 oxoéthylidène-3 bicyclooctane, dans laquelle le symbole $R_1$ représente un radical protecteur, et

a) le symbole $R_4$ représente un radical de formule générale:

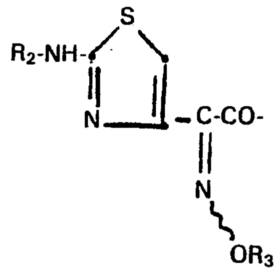

[dans laquelle $R_2$ est un radical protecteur d'amino, $R_3$ est un atome d'hydrogène, un raidcal protecteur, un radical alcoyle, vinyle, ou carboxyalcoyle représenté par la formule générale:

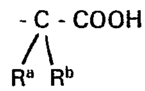

dans laquelle les radicaux $R^a$ et $R^b$, qui sont identiques ou différentes, représentent des atomes d'hydrogène ou des radicaux alcoyle, ou forment ensemble un radical alcoylène contenant 2 ou 3 atomes de carbone et dont la fonction acide peut être protégée], ou $R_4$ représente un radical $\alpha$-carboxyarylacétale libre ou protégé dans lequel aryle représente phényle, p.hydroxy phényle libre ou protégé, ou thiényle-2 ou -3, et

le symbole R'' représente un atome d'hydrogène ou un radical méthoxy en position $7\alpha$, ou bien

b) le symbole $R_4$ est un radical facilement éliminable et le symbole R'' est un atome d'hydrogène ou un radical méthoxy en $7\alpha$, ou un atome d'hydrogène en $7\beta$, étant entendu que les radicaux et portions alcoyles cités ci-dessus sont (sauf mention spéciale) droits ou ramifiés et contiennent 1 à 4 atomes de carbone, le cas échéant sous ses formes syn ou anti, caractérisé en ce que l'on hydrolyse un énamine (ou un mélange d'énamines isomères) de formule générale:

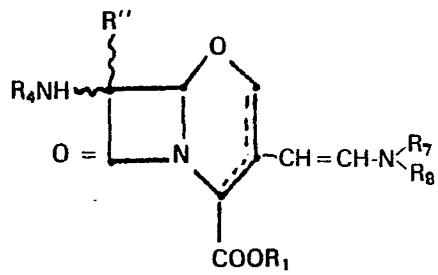

[dans laquelle $R_1$, $R_4$ et R'' sont définis comme ci-dessus et $R_7$ et $R_8$, qui sont identiques ou différents, représentent des radicaux alcoyle (éventuellement substitués par un radical alcoyloxy ou dialcoylamino) ou phényle ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle étant entendu que les radicaux et portions alcoyles cités ci-dessus sont droits ou ramifiés et contiennent 1 à 4 atomes de carbone], qui se présente sous forme bicyclooctène-2 ou -3, et dont le substituant sur l'atome de carbone en position -3 présente la stéréoisomérie E ou Z, puis on élimine éventuellement les groupements protecteurs et on sépare éventuellement les isomères du produit obtenu.

**Patentansprüche für die Vertragsstaaten:**
BE, CH, DE, FR, GB, IT, LI, LU, NL, SE

1. Ein neues Oxacephalosporin, dadurch gekennzeichnet, dass es der allgemeinen Formel

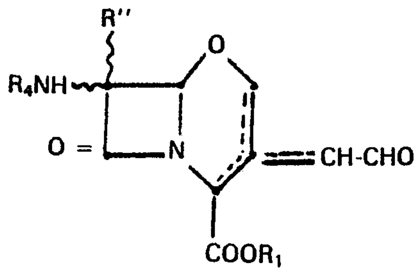

entspricht, das in Bicycloocten-2- oder -3-Form oder Oxoäthyliden-3-bicyclooctan-Form vorliegt, worin das Symbol $R_1$ einen Schutzrest bedeutet und

a) das Symbol $R_4$ einen Rest der allgemeinen Formel

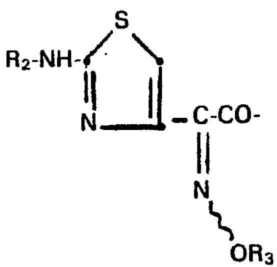

bedeutet, [worin $R_2$ ein Aminoschutzrest ist, $R_3$ ist ein Wasserstoffatom, ein Schutzrest, ein Alkyl-, Vinyl- oder Carboxyalkylrest, dargestellt durch die allgemeine Formel

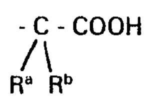

worin die Reste $R^a$ und $R^b$, die identisch oder verschieden sind, Wasserstoffatome oder Alkylreste bedeuten, oder zusammen einen Alkylenrest mit 2 oder 3 Kohlenstoffatomen bilden und deren Säurefunktion geschützt sein kann], oder $R_4$ bedeutet einen freien oder geschützten $\alpha$-Carboxyarylacetylrest, worin Aryl Phenyl, freies oder geschütztes p-Hydroxyphenyl oder Thienyl-2 oder -3 bedeutet, und

das Symbol R'' ein Wasserstoffatom oder einen Methoxyrest in $7\alpha$-Stellung bedeutet, oder

b) das Symbol $R_4$ ist ein leicht entfernbarer Rest und das Symbol R'' ist ein Wasserstoffatom oder ein Methoxyrest in $7\alpha$-Stellung oder ein Wasserstoffatom in $7\beta$-Stellung, wobei die oben erwähnten Alkylreste und -teile (wenn nichts anderes angegeben ist) gerade oder verzweigt sind und 1 bis 4 Kohlenstoffatome enthalten, gegebenenfalls in ihren syn- oder anti-Formen.

2. Ein neues Produkt gemäss Anspruch 1, dadurch gekennzeichnet, dass $R_4$ einen Rest der allgemeinen Formel

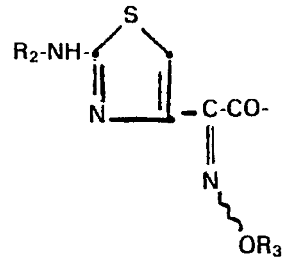

bedeutet, worin $R_3$ gemäss Anspruch 1 definiert ist und $R_2$ eine Gruppe tert.Butoxycarbonyl, 2,2,2-Trichlorethoxycarbonyl, Trichloracetyl, Chloracetyl, Trityl, Dibenzyl, Benzyl, Benzyloxycarbonyl, p-Nitrobenzyloxycarbonyl, p-Methoxybenzyloxycarbonyl, Formyl oder Trifluoracetyl ist.

3. Ein neues Produkt gemäss Anspruch 1, dadurch gekennzeichnet, dass $R_4$ einen Rest der allgemeinen Formel

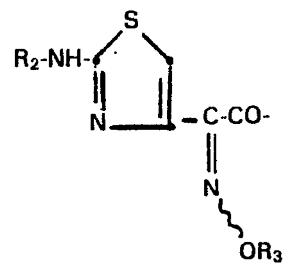

bedeutet, worin $R_2$ wie in Anspruch 1 definiert ist und $R_3$ eine Trityl-, Tetrahydropyranyl- oder 2-Methoxypropyl-2-Schutzgruppe der Oximfunktion ist.

4. Neues Produkt gemäss Anspruch 1, dadurch gekennzeichnet, dass $R_4$ ein Rest der allgemeinen Formel

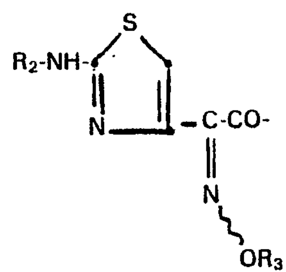

ist, worin $R_2$ wie in Anspruch 1 definiert ist und $R_3$ eine Carboxyalkylgruppe [dargestellt durch die allgemeine Formel

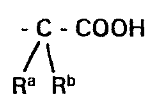

worin $R^a$ und $R^b$ wie in Anspruch 1 definiert sind] ist, die vorher durch einen Rest, ausgewählt unter Methoxymethyl, tert.Butyl, Benzhydryl, Benzyl, Nitrobenzyl und p.Methoxybenzyl, geschützt wurde.

5. Ein neues Produkt gemäss Anspruch 1, dadurch gekennzeichnet, dass $R_1$ ein leicht entfernbarer Rest ist, ausgewählt unter den Resten Methoxymethyl, tert.Butyl, Benzhydryl, Benzyl, Nitrobenzyl und p-Methoxybenzyl.

6. Verfahren zur Herstellung eines Produkts gemäss Anspruch 1, dadurch gekennzeichnet, dass man ein Enamin (oder ein Gemisch isomerer Enamine) der allgemeinen Formel

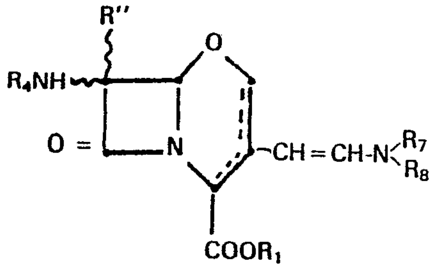

[worin $R_1$, $R_4$ und R'' wie in Anspruch 1 definiert sind und $R_7$ und $R_8$, die identisch oder verschieden sind, Alkylreste (gegebenenfalls substituiert durch einen Alkyloxy- oder Dialkylaminorest) oder Phenyl bedeuten, oder zusammen mit dem Stickstoffatom, an das sie gebundenn sind, einen gesättigten Heterozyklus mit 5 oder 6 Kettengliedern bilden, der gegebenenfalls ein weiteres Heteroatom, ausgewählt unter Stickstoff, Sauerstoff oder Schwefel, enthält und gegebenenfalls durch einen Alkylrest substituiert ist, wobei die oben erwähnten Alkylreste und -teile gerade oder verzweigt sind und 1 bis 4 Kohlenstoffatome enthalten], das in Bicycloocten-2- oder -3-Form vorliegt und dessen Substituent am Kohlenstoffatom in 3-Stellung die E- oder Z-Stereoisomerie aufweist, hydrolysiert und dass man dann gegebenenfalls die Schutzgruppen entfernt und gegebenenfalls die Isomeren des erhaltenen Produkts trennt.

**Patentanspruch für den Vertragsstaat:** AT

Verfahren zur Herstellung eines neuen Oxacephalosporins der allgemeinen Formel

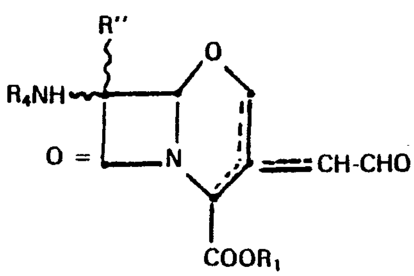

welches in 2- oder 3-Bicycloocten- oder 3-Oxoäthyliden-bicyclooctan-Form vorliegt, worin das Symbol $R_1$ eine Schutzgruppe darstellt und

a) das Symbol $R_4$ einen Rest der allgemeinen Formel

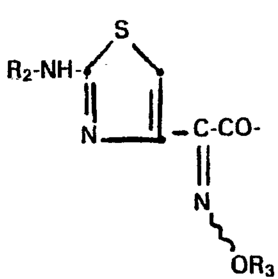

[worin $R_2$ eine Aminoschutzgruppe ist, $R_3$ ein Wasserstoffatom, eine Schutzgruppe, ein Alkyl- oder Vinylrest oder ein Carboxyalkylrest der allgemeinen Formel

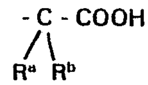

ist, worin die Reste $R^a$ und $R^b$, die gleich oder voneinander verschieden sind, Wasserstoffatome oder Alkylreste darstellen oder zusammen einen Alkylenrest mit 2 oder 3 Kohlenstoffatomen bilden, wobei die Säurefunktion geschützt sein kann], oder $R_4$ einen $\alpha$-Carboxyarylacetylrest (frei oder geschützt) darstellt, worin Aryl für Phenyl-, p-Hydroxyphenyl (frei oder geschützt) oder 2- oder 3-Thienyl steht, und das Symbol R'' ein Wasserstoffatom oder einen Methoxyrest in $7\alpha$-Stellung darstellt oder

b) das Symbol $R_4$ ein leicht entfernbarer Rest ist und das Symbol R'' ein Wasserstoffatom oder ein Methoxyrest in $7\alpha$ oder ein Wasserstoffatom in $7\beta$ ist, wobei die oben erwähnten Alkylreste und -teile (ohne besonderen Hinweis) selbstverständlich geradkettig oder verzweigt sind und 1 bis 4 Kohlenstoffatome enthalten, gegebenenfalls in den syn- oder anti-Formen, dadurch gekennzeichnet, dass man ein Enamin (oder eine Mischung isomerer Enamine) der allgemeinen Formel

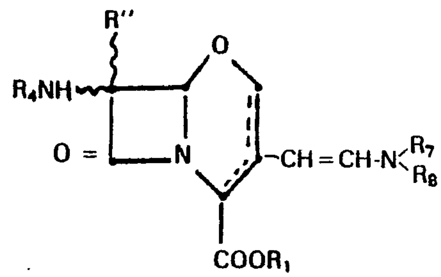

[worin $R_1$, $R_4$ und R'' die obige Bedeutung haben und $R_7$ und $R_8$, die gleich oder voneinander verschieden sind, Alkylreste (gegebenenfalls substituiert durch einen Alkyloxy- oder Dialkylaminorest) oder Phenylrest darstellen oder zusammen mit dem Stickstoffatom, an das sie gebunden sind, einen gesättigten Heterocyclus mit 5 oder 6 Gliedern bilden, der gegebenenfalls ein weiteres Heteroatom, ausgewählt aus Stickstoff, Sauerstoff und Schwefel, enthält und gegebenenfalls substituiert ist durch einen Alkylrest, wobei die oben erwähnten Alkylreste und -teile selbstverständlich geradkettig oder verzweigt sind und 1 bis 4 Kohlenstoffatome enthalten], das in 2- oder 3-Bicycloocten-Form vorliegt und dessen Substituent am in 3-Stellung befindlichen Kohlenstoffatom E- oder Z-Stereoisomerie aufweist, hydrolisiert und dann gegebenenfalls die Schutzgruppen entfernt und gegebenenfalls die Isomeren des erhaltenen Produkts trennt.

**Claims for the Contracting States:**
BE, CH, DE, FR, GB, IT, LI, LU, NL, SE

1. A new oxacephalosporin characterised by corresponding to the general formula:

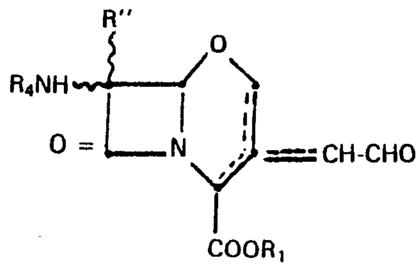

which is in the form of a bicyclooct-2-ene or bicyclo-oct-3-ene or 3-oxoethylidenebicyclooctane, in which the symbol $R_1$ denotes a protective radical, and

a) the symbol $R_4$ denotes a radical of the general formula:

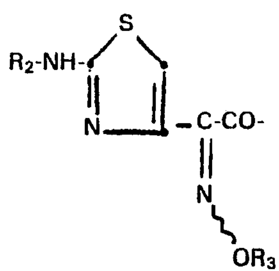

[in which $R_2$ is a protective radical for an amino group, $R_3$ is a hydrogen atom, a protective radical, an alkyl or vinyl radical, or a carboxyalkyl radical represented by the general formula:

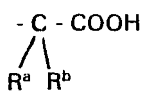

in which the radicals $R^a$ and $R^b$, which are identical or different, denote hydrogen atoms or alkyl radicals, or together form an alkylene radical containing 2 or 3 carbon atoms and whose acid function can be protected], or $R_4$ denotes a free or protected $\alpha$-carboxy-arylacetyl radical in which aryl denotes phenyl, free or protected p-hydroxyphenyl, or 2- or 3-thienyl, and the symbol R'' denotes a hydrogen atom or a methoxy radical in the 7$\alpha$-position, or else

b) the symbol $R_4$ is a radical which is easily removable and the symbol R'' is a hydrogen atom or a methoxy radical in the 7$\alpha$-position, it being understood that the abovementioned alkyl radicals and segments are (unless specially mentioned) straight-chain or branched-chain and contain 1 to 4 carbon atoms, in its syn- or anti-form where applicable.

2. A new product according to claim 1, characterised in that $R_4$ denotes a radical of the general formula:

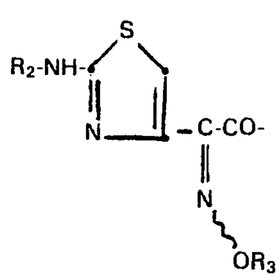

in which $R_3$ is defined according to claim 1, and $R_2$ is a t-butoxycarbonyl, 2,2,2-trichloroethoxycarbonyl, trichloroacetyl, chloroacetyl, trityl, dibenzyl, benzyl, benzyloxycarbonyl, p-nitrobenzyloxycarbonyl, p-methoxybenzyloxycarbonyl, formyl or trifluoroacetyl group.

3. A new product according to claim 1, characterised in that $R_4$ denotes a radical of the general formula:

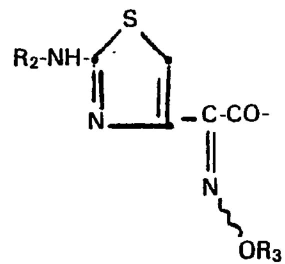

in which $R_2$ is defined as in claim 1, and $R_3$ is a trityl, tetrahydropyrannyl, or 2-methoxyprop-2-yl group for protecting the oxime group.

4. A new product according to claim 1, characterised in that $R_4$ is a radical of the general formula:

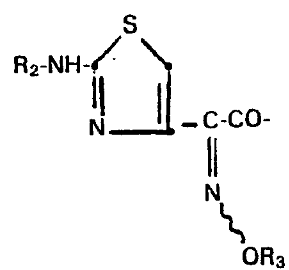

in which $R_2$ is defined as in claim 1, and $R_3$ is a carbonylalkyl group [represented by the general formula:

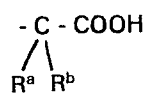

in which $R^a$ and $R^b$ are defined as in claim 1] which is protected beforehand with a radical chosen from the methoxymethyl, t-butyl, benzhydryl, benzyl, nitrobenzyl and p-methoxybenzyl radicals.

5. A new product according to claim 1, characterised in that $R_1$ is an easily removable radical chosen from the methoxymethyl, t-butyl, benzhydryl, benzyl, nitrobenzyl and p-methoxybenzyl radicals.

6. A process for preparing a product according to claim 1, characterised by carrying out the hydrolysis of an enamine (or a mixture of isomeric enamines) of the general formula:

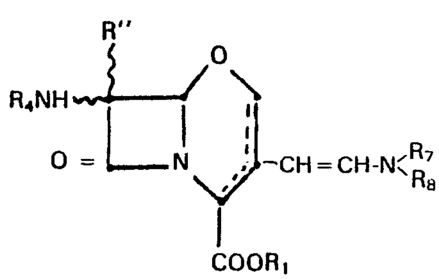

[in which $R_1$, $R_4$ and R'' are defined as in claim 1 and $R_7$ and $R_8$, which are identical or different, denote alkyl radicals (optionally substituted with an alkoxy or dialkylamino radical) or phenyl radicals, or form, together with the nitrogen atom to which they are attached, a saturated 5- or 6-membered hererocyclic ring optionally containing another heteroatom chosen from nitrogen, oxygen and sulphur, and optionally substituted with an alkyl radical, it being understood that the abovementioned alkyl radicals and segments are straight-chain or branched-chain and contain 1 to 4 carbon atoms], which is in the form of a bicyclooct-2-ene or bicyclooct-3-ene, and in which the substituent on the carbon atom in the 3-position has the stereoisomeric configuration E or Z, and then removing the protective groups, if appropriate, and separating, if appropriate, the isomers of the product which is obtained.

**Claim for the Contracting State:** AT

A process for the preparation of a new oxacephalosporin of the general formula:

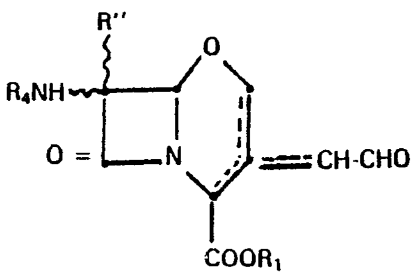

which is in the form of a bicyclooct-2-ene or bicyclooct-3-ene or 3-oxoethylidenebicyclooctane, in which the symbol $R_1$ denotes a protective radical, and

a) the symbol $R_4$ denotes a radical of the general formula:

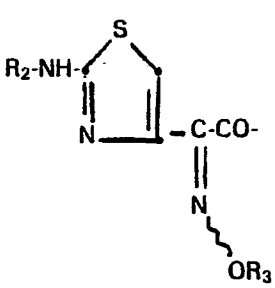

[in which $R_2$ is a protective radical for an amino group, $R_3$ is a hydrogen atom, a protective radical, an alkyl or vinyl radical, or a carboxyalkyl radical represented by the general formula:

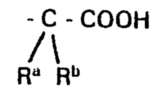

in which the radicals $R^a$ and $R^b$, which are identical or different, denote hydrogen atoms or alkyl radicals, or together form an alkylene radical containing 2 or 3 carbon atoms and whose acid function can be protected], or $R_4$ denotes a free or protected $\alpha$-carboxyarylacetyl radical in which aryl denotes phenyl, free or protected p-hydroxyphenyl, or 2- or 3-thienyl, and the symbol R'' denotes a hydrogen atom or a methoxy radical in the $7\alpha$-position, or b) the symbol $R_4$ is a radical which is easily removable and the symbol R'' is a hydrogen atom or a methoxy radical in the $7\alpha$-position, or a hydrogen atom in the $7\beta$-position, it being understood that the abovementioned alkyl radicals and segments are (unless specially mentioned) straight-chain or branched-chain and contain 1 to 4 carbon atoms, in its syn- or anti- form where applicable, characterised by carrying out the hydrolysis of an enamine (or a mixture of isomeric enamines) of the general formula:

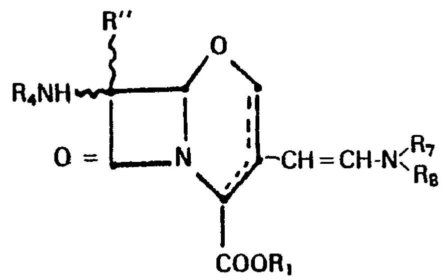

[in which $R_1$, $R_4$ and R'' are defined as above and $R_7$ and $R_8$, which are identical or different, denote alkyl radicals (optionally with an alkoxy or dialkylamino radical) or phenyl radicals, or form, together with the nitrogen atom to which they are attached, a saturated 5- or 6-membered heterocyclic ring optionally containing another heteroatom chosen from nitrogen, oxygen and sulphur, and optionally substituted with an alkyl radical, it being understood that the abovementioned alkyl radicals and segments are straight-chain or branched-chain and contain 1 to 4 carbon atoms], which is in the form of a bicyclooct-2-ene or bicyclooct-3-ene, and in which the substituent on the carbon atom in the 3-position has the stereoisomeric configuration E or Z, and then removing the protective groups, if appropriate, and separating, if appropriate, the isomers of the product which is obtained.